# EUROPEAN PATENT SPECIFICATION

(11) **EP 0 990 000 B1**
(45) Date of publication and mention of the grant of the patent: **27.07.2005**
(21) Application number: 96938816.4
(22) Date of filing: 08.11.1996
(51) Int. Cl.: C07K 14/475, C12P 21/06, C12N 5/00, C12N 15/00, A01N 37/18, C07H 17/00

(54) **CONNECTIVE TISSUE GROWTH FACTOR-3**
BINDEGEWEBE-WACHSTUMSFAKTOR 3.
FACTEUR DE CROISSANCE 3 DU TISSU CONJONCTIF

(43) Date of publication of application: 05.04.2000
(73) Proprietor: Human Genome Sciences, Inc., Rockville, MD 20850 (US)
(72) Inventor: EBNER, Reinhard, Gaithersburg, MD 20878 (US); CHOPRA, Arvind, Gaithersburg, MD 20878 (US); RUBIN, Steven, M., Brookville, MD 20833 (US)
(74) Representative: VOSSIUS & PARTNER
(86) International application number: PCT/US1996/017856
(87) International publication number: WO 1998/021236

(56) References cited:
- WO-A-96/38168
- US-A- 5 408 040
- J. INVEST. DERMATOL., 1996, Vol. 106, No. 4, IGARASHI et al., "Connective Tissue Growth Factor Gene Expression in Tissue Sections from Localized Scleroderma, Keloid and Other Fibrotic Skin Disorders", pages 729-733.
- FEBS, July 1993, Vol. 327, No. 2, BORK P., "The Modular Architecture of a New Family of Growth Regulators Related to Connective Tissue Growth Factor", pages 125-130.
- J. CELL. BIOL., September 1991, Vol. 114, No. 6, BRADHAM et al., "Connective Tissue Growth Factor: A Cysteine-Rich Mitoen Secreted by Human Vascular Endothelial Cells is Related to the SRC-Induced Immediate Early Gene Product CEF-10", pages 1285-1294.
- J. CLIN. PATHOL., 1996, Vol. 49, XIN et al., "Differential Expression of novH and CTGF in Human Glioma Cell Lines", pages M91-M97.

### SEQUENCE LISTING

(1) GENERAL INFORMATION:
   (i) APPLICANT: Human Genome Sciences, Inc. 9410 Key West Avenue Rockville, MD 20850 United States of America
      APPLICANTS/INVENTORS: Ebner, Reinhard Chopra, Arvind Ruben, Steven M.
   (ii) TITLE OF INVENTION: Connective Tissue Growth Factor-3
   (iii) NUMBER OF SEQUENCES: 9
   (iv) CORRESPONDENCE ADDRESS:
      (A) ADDRESSEE: Sterne, Kessler, Goldstein & Fox, P.L.L.C.
      (B) STREET: 1100 New York Ave, NW, Suite 600
      (C) CITY: Washington
      (D) STATE: DC
      (E) COUNTRY: USA
      (F) ZIP: 20005-3934
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: To be assigned
      (B) FILING DATE: Herewith
      (C) CLASSIFICATION:
   (viii) ATTORNEY/AGENT INFORMATION:
      (A) NAME: Goldstein, Jorge A.
      (B) REGISTRATION NUMBER: 29,021
      (C) REFERENCE/DOCKET NUMBER: 1488.063PC00/JAG/EKS/KRM
   (ix) TELECOMMUNICATION INFORMATION:
      (A) TELEPHONE: 202-371-2600
      (B) TELEFAX: 202-371-2543
(2) INFORMATION FOR SEQ ID NO:1:
   (i) SEQUENCE CHARACTERISTICS:
      (A) LENGTH: 1285 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 9..758
   (ix) FEATURE:
      (A) NAME/KEY: sig_peptide
      (B) LOCATION: 9..65
   (ix) FEATURE:
      (A) NAME/KEY: mat_peptide
      (B) LOCATION: 66..758
   (xi) SEQUENCE DESCRIPTION: SEQ ID NO:1:
   CAGGGGAC ATG AGA GGC ACA CCG AAG ACC CAC CTC CTG GCC TTC TCC CTC       50
            Met Arg Gly Thr Pro Lys Thr His Leu Leu Ala Phe Ser Leu
            -19             -15                     -10
   CTC TGC CTC CTC TCA AAG GTG CGT ACC CAG CTG TGC CCG ACA CCA TGT        98
   Leu Cys Leu Leu Ser Lys Val Arg Thr Gln Leu Cys Pro Thr Pro Cys
    -5                     1               5                  10
   ACC TGC CCC TGG CCA CCT CCC CGA TGC CCG CTG GGA GTA CCC CTG GTG       146
   Thr Cys Pro Trp Pro Pro Pro Arg Cys Pro Leu Gly Val Pro Leu Val
               15                  20                  25
   CTG GAT GGC TGT GGC TGC TGC CGG GTA TGT GCA CGG CGG CTG GGG GAG       194
   Leu Asp Gly Cys Gly Cys Cys Arg Val Cys Ala Arg Arg Leu Gly Glu
           30                  35                  40
   CCC TGC GAC CAA CTC CAC GTC TGC GAC GCC AGC CAG GGC CTG GTC TGC       242
   Pro Cys Asp Gln Leu His Val Cys Asp Ala Ser Gln Gly Leu Val Cys
       45                  50                  55
   CAG CCC GGG GCA GGA CCC GGT GGC CGG GGG GCC CTG TGC CTC TTG GCA       290
   Gln Pro Gly Ala Gly Pro Gly Gly Arg Gly Ala Leu Cys Leu Leu Ala
    60                  65                  70                  75
   GAG GAC GAC AGC AGC TGT GAG GTG AAC GGC CGC CTG TAT CGG GAA GGG       338
   Glu Asp Asp Ser Ser Cys Glu Val Asn Gly Arg Leu Tyr Arg Glu Gly
                   80                  85                  90
   GAG ACC TTC CAG CCC CAC TGC AGC ATC CGC TGC CGC TGC GAG GAC GGC       386
   Glu Thr Phe Gln Pro His Cys Ser Ile Arg Cys Arg Cys Glu Asp Gly
               95                  100                 105
   GGC TTC ACC TGC GTG CCG CTG TGC AGC GAG GAT GTG CGG CTG CCC AGC       434
   Gly Phe Thr Cys Val Pro Leu Cys Ser Glu Asp Val Arg Leu Pro Ser
           110                 115                 120
   TGG GAC TGC CCC CAC CCC AGG AGG GTC GAG GTC CTG GGC AAG TGC TGC       482
   Trp Asp Cys Pro His Pro Arg Arg Val Glu Val Leu Gly Lys Cys Cys
       125                 130                 135
   CCT GAG TGG GTG TGC GGC CAA GGA GGG GGA CTG GGG ACC CAG CCC CTT       530
   Pro Glu Trp Val Cys Gly Gln Gly Gly Gly Leu Gly Thr Gln Pro Leu
   140                 145                 150                 155
   CCA GCC CAA GGA CCC CAG TTT TCT GGC CTT GTC TCT TCC CTG CCC CCT       578
   Pro Ala Gln Gly Pro Gln Phe Ser Gly Leu Val Ser Ser Leu Pro Pro
                   160                 165                 170
   GGT GTC CCC TGC CCA GAA TGG AGC ACG GCC TGG GGA CCC TGC TCG ACC       626
   Gly Val Pro Cys Pro Glu Trp Ser Thr Ala Trp Gly Pro Cys Ser Thr
               175                 180                 185
   ACC TGT GGG CTG GGC ATG GCC ACC CGG GTG TCC AAC CAG AAC CGC TTC       674
   Thr Cys Gly Leu Gly Met Ala Thr Arg Val Ser Asn Gln Asn Arg Phe
           190                 195                 200
   TGC CGA CTG GAG ACC CAG CGC CGC CTG TGC CTG TCC AGG CCC TGC CCA       722
   Cys Arg Leu Glu Thr Gln Arg Arg Leu Cys Leu Ser Arg Pro Cys Pro
       205                 210                 215
   CCC TCC AGG GGT CGC AGT CCA CAA AAC AGT GCC TTC TAGAGCCGGG            768
   Pro Ser Arg Gly Arg Ser Pro Gln Asn Ser Ala Phe
   220                 225                 230
   CTGGGAATGG GGACACGGTG TCCACCATCC CCAGCTGGTG GCCCTGTGCC TGGGCCCTGG     828
   GCTGATGGAA GATGGTCCGT GCCCAGGCCC TTGGCTGCAG GCAACACTTT AGCTTGGGTC     888
   CACCATGCAG AACACCAATA TTAACACGCT GCCTGGTCTG TCTGGATCCC GAGGTATGGC     948
   AGAGGTGCAA GACCTAGTCC CCTTTCCTCT AACTCACTGC CTAGGAGGCT GGCCAAGGTG    1008
   TCCAGGGTCC TCTAGCCCAC TCCCTGCCTA CACACACAGC CTATATCAAA CATGCACACG    1068
   GGCGAGCTTT CTCTCCGACT TCCCCTGGGC AAGAGATGGG ACAAGCAGTC CCTTAATATT    1128
   GAGGCTGCAG CAGGTGCTGG GCTGGACTGG CCATTTTTCT GGGGGTAGGA TGAAGAGAAG    1188
   GCACACAGAG ATTCTGGATC TCCTGCTGCC TTTTCTGGAG TTTGTAAAAT TGTTCCTGAA    1248
   TACAAGCCTA TGCGTGAAAA AAAAAAAAAA AAAAAAA                             1285
(2) INFORMATION FOR SEQ ID NO:2:
   (i) SEQUENCE CHARACTERISTICS:
      (A) LENGTH: 250 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Arg Gly Thr Pro Lys Thr His Leu Leu Ala Phe Ser Leu Leu Cys
   -19             -15                  -10                  -5
   Leu Leu Ser Lys Val Arg Thr Gln Leu Cys Pro Thr Pro Cys Thr Cys
                1               5                  10
   Pro Trp Pro Pro Pro Arg Cys Pro Leu Gly Val Pro Leu Val Leu Asp
        15                  20                  25
   Gly Cys Gly Cys Cys Arg Val Cys Ala Arg Arg Leu Gly Glu Pro Cys
    30                  35                  40                  45
   Asp Gln Leu His Val Cys Asp Ala Ser Gln Gly Leu Val Cys Gln Pro
                    50                  55                  60
   Gly Ala Gly Pro Gly Gly Arg Gly Ala Leu Cys Leu Leu Ala Glu Asp
                65                  70                  75
   Asp Ser Ser Cys Glu Val Asn Gly Arg Leu Tyr Arg Glu Gly Glu Thr
            80                  85                  90
   Phe Gln Pro His Cys Ser Ile Arg Cys Arg Cys Glu Asp Gly Gly Phe
        95                 100                 105
   Thr Cys Val Pro Leu Cys Ser Glu Asp Val Arg Leu Pro Ser Trp Asp
   110                 115                 120                 125
   Cys Pro His Pro Arg Arg Val Glu Val Leu Gly Lys Cys Cys Pro Glu
                   130                 135                 140
   Trp Val Cys Gly Gln Gly Gly Gly Leu Gly Thr Gln Pro Leu Pro Ala
               145                 150                 155
   Gln Gly Pro Gln Phe Ser Gly Leu Val Ser Ser Leu Pro Pro Gly Val
           160                 165                 170
   Pro Cys Pro Glu Trp Ser Thr Ala Trp Gly Pro Cys Ser Thr Thr Cys
       175                 180                 185
   Gly Leu Gly Met Ala Thr Arg Val Ser Asn Gln Asn Arg Phe Cys Arg
   190                 195                 200                 205
   Leu Glu Thr Gln Arg Arg Leu Cys Leu Ser Arg Pro Cys Pro Pro Ser
                   210                 215                 220
   Arg Gly Arg Ser Pro Gln Asn Ser Ala Phe
               225                 230
(2) INFORMATION FOR SEQ ID NO:3:
   (i) SEQUENCE CHARACTERISTICS:
      (A) LENGTH: 349 amino acids
      (B) TYPE: amino acid
      (C) STRANDEDNESS: not relevant
      (D) TOPOLOGY: not relevant
   (ii) MOLECULE TYPE: protein
   (xi) SEQUENCE DESCRIPTION: SEQ ID NO:3:
   Met Thr Ala Ala Ser Met Gly Pro Val Arg Val Ala Phe Val Val Leu
   1               5                   10                  15
   Leu Ala Leu Cys Ser Arg Pro Ala Val Gly Gln Asn Cys Ser Gly Pro
               20                  25                  30
   Cys Arg Cys Pro Asp Glu Pro Ala Pro Arg Cys Pro Ala Gly Val Ser
           35                  40                  45
   Leu Val Leu Asp Gly Cys Gly Cys Cys Arg Val Cys Ala Lys Gln Leu
       50                  55                  60
   Gly Glu Leu Cys Thr Glu Arg Asp Pro Cys Asp Pro His Lys Gly Leu
   65                  70                  75                  80
   Phe Cys Asp Phe Gly Ser Pro Ala Asn Arg Lys Ile Gly Val Cys Thr
                   85                  90                  95
   Ala Lys Asp Gly Ala Pro Cys Ile Phe Gly Gly Thr Val Tyr Arg Ser
               100                 105                 110
   Gly Glu Ser Phe Gln Ser Ser Cys Lys Tyr Gln Cys Thr Cys Leu Asp
           115                 120                 125
   Gly Ala Val Gly Cys Met Pro Leu Cys Ser Met Asp Val Arg Leu Pro
       130                 135                 140
   Ser Pro Asp Cys Pro Phe Pro Arg Arg Val Lys Leu Pro Gly Lys Cys
   145                 150                 155                 160
   Cys Glu Glu Trp Val Cys Asp Glu Pro Lys Asp Gln Thr Val Val Gly
                   165                 170                 175
   Pro Ala Leu Ala Ala Tyr Arg Leu Glu Asp Thr Phe Gly Pro Asp Pro
               180                 185                 190
   Thr Met Ile Arg Ala Asn Cys Leu Val Gln Thr Thr Glu Trp Ser Ala
           195                 200                 205
   Cys Ser Lys Thr Cys Gly Met Gly Ile Ser Thr Arg Val Thr Asn Asp
       210                 215                 220
   Asn Ala Ser Cys Arg Leu Glu Lys Gln Ser Arg Leu Cys Met Val Arg
   225                 230                 235                 240
   Pro Cys Glu Ala Asp Leu Glu Glu Asn Ile Lys Lys Gly Lys Lys Cys
                   245                 250                 255
   Ile Arg Thr Pro Lys Ile Ser Lys Pro Ile Lys Phe Glu Leu Ser Gly
               260                 265                 270
   Cys Thr Ser Met Lys Thr Tyr Arg Ala Lys Phe Cys Gly Val Cys Thr
           275                 280                 285
   Asp Gly Arg Cys Cys Thr Pro His Arg Thr Thr Thr Leu Pro Val Glu
       290                 295                 300
   Phe Lys Cys Pro Asp Gly Glu Val Met Lys Lys Asn Met Met Phe Ile
   305                 310                 315                 320
   Lys Thr Cys Ala Cys His Tyr Asn Cys Pro Gly Asp Asn Asp Ile Phe
                   325                 330                 335
   Glu Ser Leu Tyr Tyr Arg Lys Met Tyr Gly Asp Met Ala
               340                 345
(2) INFORMATION FOR SEQ ID NO:4:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   CACCACGGAT CCAAGGTGCG TACCCAGCTG TGCCCG                36
(2) INFORMATION FOR SEQ ID NO:5:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   GATGTAAGCT TCGTGTCCCC ATTCCCAGCC CG                    32
(2) INFORMATION FOR SEQ ID NO:6:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
   CGGCAGGATC CGCCATCATG AGAGGCACAC CGAAGACCC 39
(2) INFORMATION FOR SEQ ID NO:7:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
   GATGTGGTAC CCGTGTCCCC ATTCCCAGCC CG 32
(2) INFORMATION FOR SEQ ID NO:8:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:8:
   GCTCGGATCC GCCATCATGA GAGGCACACC GAAGACCCAC 40
(2) INFORMATION FOR SEQ ID NO:9:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   GATGTTCTAG AAGAAGGCAC TGTTTTGTGG ACTGCGACCC CTG                          43

## Description

### Background of the Invention

### Field of the Invention

The present invention relates to a novel connective tissue growth factor. More specifically, isolated nucleic acid molecules are provided encoding a human connective tissue growth factor-3. Connective tissue growth factor-3 polypeptides are also provided, as are vectors, host cells, and recombinant methods for producing the same. Also provided are diagnostic and therapeutic methods for detecting osteoporosis and uses for treating osteoporosis.

### Related Art

Growth factors are a class of secreted cysteine-rich polypeptides that stimulate target cells to proliferate, differentiate, and organize in developing tissues. The action of growth factors is dependent on their binding to specific receptors, which stimulate a signaling event within the cell. Examples of some well-studied growth factors include platelet-derived growth factor (PDGF), insulin-like growth factor (IGF-I), transforming growth factor beta (TGF-β), transforming growth factor alpha (TGF-α), epidermal growth factor (EGF), and fibroblast growth factor (FGF). This group of growth factors is important for normal growth, differentiation, morphogenesis of the cartilaginous skeleton of an embryo, and cell growth. Among some of the functions that have been reported for these growth factors are wound healing, tissue repair/regeneration, implant fixation, and stimulating bone mass increase.

PDGF is a cationic, heat-stable protein found in the alpha-granules of circulating platelets and is known to be a mitogen and chemotactic agent for connective tissue cells such as fibroblasts and smooth muscle cells. Because of the activities of this molecule, PDGF is believed to be a major factor involved in the normal healing of wounds and pathologically contributes to such diseases as atherosclerosis and fibrotic diseases. PDGF is a dimeric molecule consisting of an A chain and a B chain. The chains form heterodimers or homodimers and all combinations isolated to date are biologically active.

Studies on the role of various growth factors in tissue regeneration and repair have led to the discovery of PDGF-like proteins. These proteins share both immunological and biological activities with PDGF and can be blocked with antibodies specific to PDGF.

U.S. Patent 5,408,040 to Grotendorst *et al.* (1995) discloses a PDGF-like protein called Connective Tissue Growth Factor (CTGF) that reportedly plays a significant role in the normal development, growth, and repair of human tissue. The discovery of the CTGF protein and the cloning of the cDNA encoding the protein was reportedly significant in that it was a previously unknown growth factor having mitogenic and chemotactic activities for connective tissue cells. Although the biological activity of CTGF was similar to that of PDGF, CTGF is the product of a gene unrelated to the A or B chain genes of PDGF.

Since CTGF is produced by endothelial and fibroblastic cells, both of which are present at the site of a wound, it is probable that CTGF functions as a growth factor in wound healing. Accordingly, it is believed that the CTGF polypeptide could be used as a therapeutic in cases in which there is impaired healing of skin wounds or where there is a need to augment the normal healing process.

Pathologically, CTGF may also be involved in diseases in which there is an overgrowth of connective tissue cells or an enhanced production of extracellular matrix components. Such diseases include cancer, fibrosis, and atherosclerosis. For example, it has been shown that CTGF gene expression is elevated in the skin of patients with systemic sclerosis (SSc). Igarashi *et al., J Invest. Dermatol. 105*:280-284 (1995). CTGF gene expression has also recently been demonstrated in several fibrotic skin diseases, such as localized scleroderma, keloid scars, nodular fasciitis, and eosinophilic fasciitis, suggesting a pathogenic role for this molecule in skin fibrosis. Igarashi *et al., J Invest. Dermatol. 106*:729-733 (1996). Oemar *et al., Circulation 92*(8), Supp't 1, Abstract 0811 (Oct. 1995) have reported that human CTGF is expressed at 5-10 fold higher levels in the aorta, a tissue prone to develop atherosclerosis, as compared to expression levels in internal mammary arteries, which are resistant to artherosclerosis. Their results suggest that hCTGF may play an essential role in the development and progression of atherosclerosis. Therapeutically, it has been reported in U.S. Patent 5,408,040 to Grotendorst *et al*. (1995) that CTGF antibodies or fragments of the antibody could be used to neutralize the biological activity of CTGF in diseases where CTGF is inducing the overgrowth of tissue. Additionally, antibodies to CTGF polypeptide or fragments could be valuable as diagnostic tools to aid in the detection of diseases in which CTGF is a pathological factor. *Id*.

Due to the important role of CTGF in the development and repair of human tissue, as well as its role in the development and progression of various connective-tissue related disorders, there is a clear need in the art for the identification of new connective tissue growth factors that can be utilized in the development of diagnostics and therapeutics for various connective tissue related disorders.

### Summary of the Invention

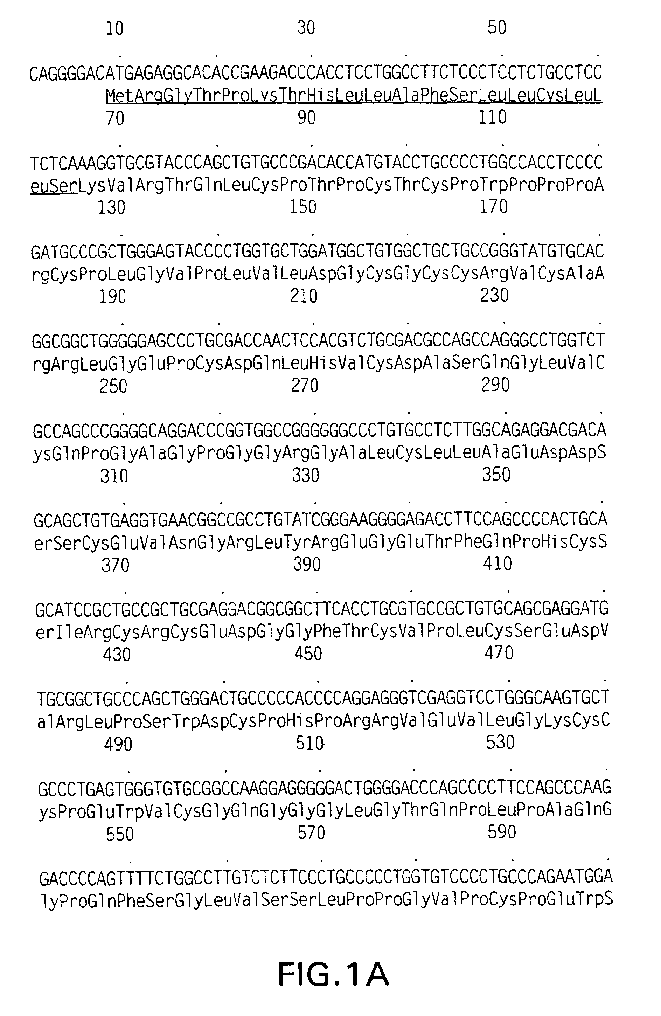

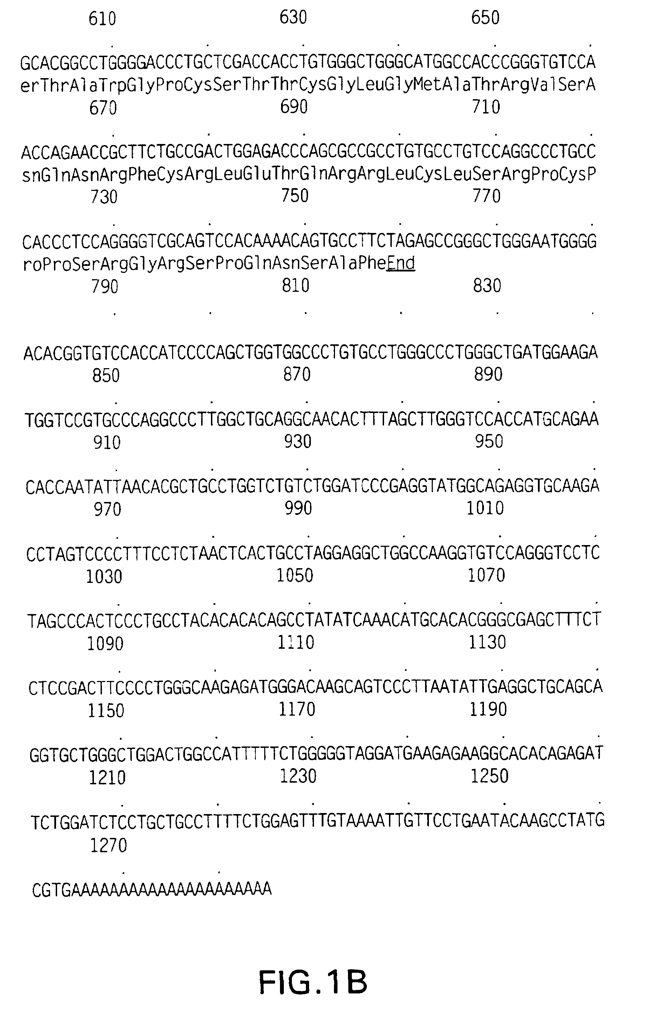

The present invention provides polynucleotides encoding the connective tissue growth factor-3 polypeptide having the amino acid sequence shown in Figure 1 (SEQ ID NO:2) or the amino acid sequence encoded by the cDNA clone deposited in a bacterial host as ATCC Deposit Number 97756 on October 10, 1996. The nucleotide sequence determined by sequencing the deposited connective tissue growth factor-3 clone, which is shown in Figure 1 (SEQ ID NO:1), contains an open reading frame encoding a polypeptide of 250 amino acid residues, including an initiation codon at positions 17-19, with a leader sequence of about 19 amino acid residues, and a predicted molecular weight of about 26 kDa. The amino acid sequence of the mature connective tissue growth factor-3 protein is shown in Figure 1, amino acid residues 20 to 250 (SEQ ID NO:2).

Thus, one aspect of the invention provides a polynucleotide having a nucleotide sequence selected from the group consisting of: (a) a nucleotide sequence encoding the connective tissue growth factor-3 polypeptide having the complete amino acid sequence in Figure 1 (SEQ ID NO:2); (b) a nucleotide sequence encoding the mature connective tissue growth factor-3 polypeptide having the amino acid sequence at positions 20 to 250 in Figure 1 (SEQ ID NO:2); (c) a nucleotide sequence encoding the connective tissue growth factor-3 polypeptide having the complete amino acid sequence encoded by the cDNA clone contained in ATCC Deposit No. 97756; (d) a nucleotide sequence encoding the mature connective tissue growth factor-3 polypeptide having the amino acid sequence encoded by the cDNA clone contained in ATCC Deposit No. 97756; and (e) a nucleotide sequence complementary to any of the nucleotide sequences in (a), (b), (c), or (d) above.

Further embodiments of the invention include isolated nucleic acid molecules having a nucleotide sequence at least 95%, 96%, 97%, 98%, or 99% identical, to any of the nucleotide sequences in (a), (b), (c), (d), or (e), above, or a polynucleotide which hybridizes under stringent hybridization conditions to a polynucleotide in (a), (b), (c), (d), or (e), above and which encodes a polypeptide that promotes an increase in bone mass. This polynucleotide which hybridizes does not hybridize under stringent conditions to a polynucleotide having a nucleotide sequence consisting of only A residues or of only T residues.

The present invention also relates to recombinant vectors, which include the isolated nucleic acid molecules of the present invention, and to host cells containing the recombinant vectors, as well as to methods of making such vectors and host cells and for using them for production of connective tissue growth factor-3 polypeptides or peptides by recombinant techniques.

The invention further provides an isolated connective tissue growth factor-3 polypeptide having an amino acid sequence selected from the group consisting of: (a) the amino acid sequence of the connective tissue growth factor-3 polypeptide having the complete 250 amino acid sequence, including the leader sequence shown in Figure 1 (SEQ ID NO:2); (b) the amino acid sequence of the mature connective tissue growth factor-3 polypeptide (without the leader) having the amino acid sequence at positions 20 to 250 in Figure 1 (SEQ ID NO:2); (c) the amino acid sequence of the connective tissue growth factor-3 polypeptide having the complete amino acid sequence, including the leader, encoded by the cDNA clone contained in ATCC Deposit No. 97756; and (d) the amino acid sequence of the mature connective tissue growth factor-3 polypeptide having the amino acid sequence encoded by the cDNA clone contained in ATCC Deposit No. 97756. The polypeptides of the present invention also include polypeptides having an amino acid sequence with at least 90% similarity, and more preferably at least 95% similarity to those described in (a), (b), (c), or (d) above, as well as polypeptides having an amino acid sequence at least 95%, 96%, 97%, 98%, or 99% identical to those above.

The invention also describes a peptide or polypeptide, which has the amino acid sequence of an epitope-bearing portion of a connective tissue growth factor-3 polypeptide having an amino acid sequence described in (a), (b), (c), or (d), above. Peptides or polypeptides having the amino acid sequence of an epitope-bearing portion of a connective tissue growth factor-3 polypeptide of the invention include portions of such polypeptides with at least six or seven, preferably at least nine, and more preferably at least about 30 amino acids to about 50 amino acids, although epitope-bearing polypeptides of any length, up to and including the entire amino acid sequence of a polypeptide of the invention described above, also are described in the invention. In another embodiment, the invention provides an isolated antibody that binds specifically to a connective tissue growth factor-3 polypeptide having an amino acid sequence described in (a), (b), (c), or (d) above.

The invention further provides methods for isolating antibodies that bind specifically to a connective tissue growth factor-3 polypeptide having an amino acid sequence as described herein. Such antibodies are useful diagnostically or therapeutically as described below.

The present invention also describes a screening method for identifying compounds capable of enhancing or inhibiting a cellular response induced by connective tissue growth factor-3, which involves contacting cells that express connective tissue growth factor-3 with the candidate compound, assaying a cellular response, and comparing the cellular response to a standard cellular response, the standard being assayed when contact is made in the absence of the candidate compound; whereby, an increased cellular response over the standard indicates that the compound is an agonist and a decreased cellular response over the standard indicates that the compound is an antagonist.

In another aspect, a screening assay for agonists and antagonists is described which involves determining the effect a candidate compound has on connective tissue growth factor-3 binding to the connective tissue growth factor-3 receptor. In particular, the method involves contacting the connective tissue growth factor-3 receptor with a connective tissue growth factor-3 polypeptide and a candidate compound and determining whether connective tissue growth factor-3 polypeptide binding to the connective tissue growth factor-3 receptor is increased or decreased due to the presence of the candidate compound.

The present inventors have discovered that connective tissue growth factor-3 is expressed in multiple human tissues, including, for example, ovary, testis, heart, lung, skeletal muscle, adrenal medulla, adrenal cortex, thymus, prostate, small intestine, and colon. It is also expected thai connective tissue growth factor-3 will be expressed in fibrotic human skin and liver. For a number of connective tissue disorders or clinical states, it is believed that significantly higher or lower levels of connective tissue growth factor-3 gene expression can be detected in certain tissues (e.g., ovary, testis, fibrotic skin and liver) or bodily fluids (e.g., serum, plasma, urine, synovial fluid or spinal fluid) taken from an individual having such a disorder, relative to a "standard" connective tissue growth factor-3 gene expression level, i.e., the connective tissue growth factor-3 expression level in tissue or bodily fluids from an individual not having the connective tissue related disorder. Thus, the invention provides a diagnostic method useful during diagnosis of osteoporosis which involves: (a) assaying connective tissue growth factor-3 gene expression level in cells or body fluid of an individual; (b) comparing the connective tissue growth factor-3 gene expression level with a standard connective tissue related growth factor-3 gene expression level, whereby an increase or decrease in the assayed connective tissue growth factor-3 gene expression level compared to the standard expression level is indicative of osteoporosis.

An additional aspect of the invention is related to the use of a composition comprising a therapeutically effective amount of an isolated connective tissue growth factor-3 polypeptide of the invention for the preparation of a pharmaceutical composition for treating osteoporosis.

### Brief Description of the Figures

Figures 1A and 1B show the nucleotide (SEQ ID NO:1) and deduced amino acid (SEQ ID NO:2) sequences of connective tissue growth factor-3. The protein has a leader sequence of about 19 amino acid residues (underlined) and a deduced molecular weight of about 26 kDa. The predicted amino acid sequence of the mature connective tissue growth factor-3 protein is also shown in Figures 1A and 1B (SEQ ID NO:2).

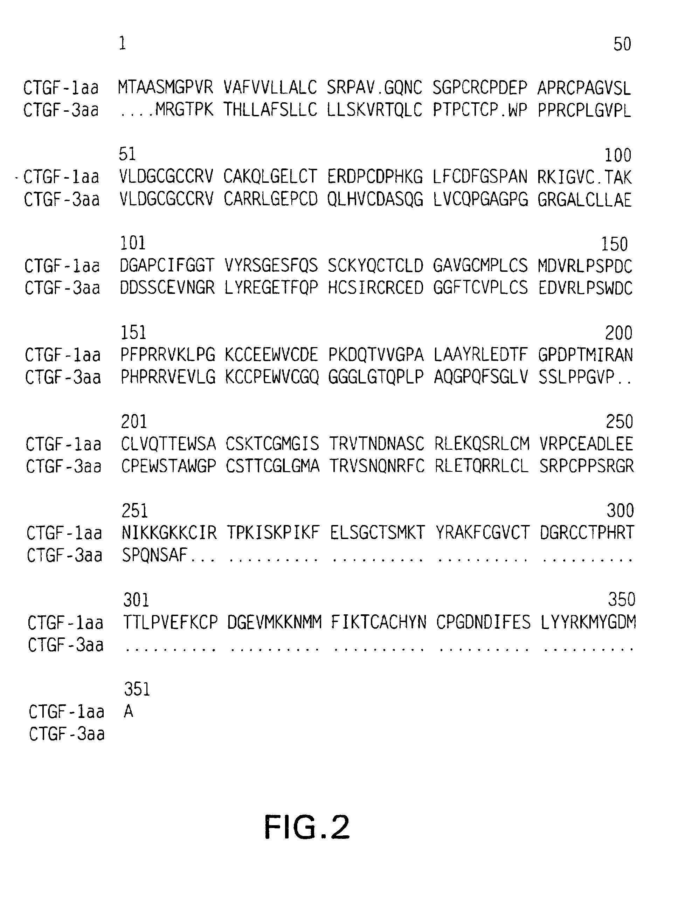

Figure 2 shows the regions of similarity between the amino acid sequences of the connective tissue growth factor-3 protein and connective tissue growth factor-1 (SEQ ID NO:3).

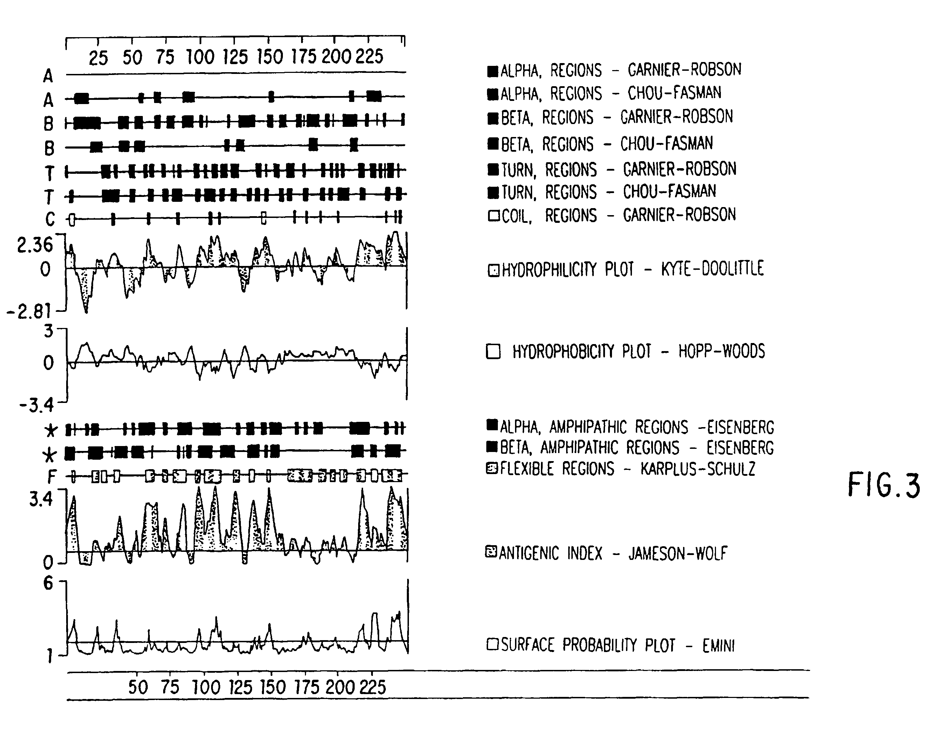

Figure 3 shows an analysis of the connective tissue growth factor-3 amino acid sequence. Alpha, beta, turn, and coil regions; hydrophilicity and hydrophobicity; amphipathic regions; flexible regions; antigenic index and surface probability are shown. In the "Antigenic Index - Jameson-Wolf" graph, amino acid residues 55-68, 94-128, 134-158, and 215-249 in Figure 1 correspond to the shown highly antigenic regions of the connective tissue growth factor-3 protein.

### Detailed Description

The present invention provides a polynucleotide encoding a connective tissue growth factor-3 polypeptide having the amino acid sequence shown in Figure 1 (SEQ ID NO:2), which was determined by sequencing a cloned cDNA. The connective tissue growth factor-3 protein of the present invention shares sequence homology with connective tissue growth factor-1 (Figure 2) (SEQ ID NO:3). The nucleotide sequence shown in Figure 1 (SEQ ID NO:1) was obtained by sequencing a cDNA clone, which was deposited October 10, 1996 at the American Type Culture Collection, 12301 Park Lawn Drive, Rockville, Maryland 20852, and given accession number 97756. The deposited clone is contained in the Uni-Zap XR vector (Stratagene, LaJolla, CA).

### Nucleic Acid Molecules

Unless otherwise indicated, all nucleotide sequences determined by sequencing a DNA molecule herein were determined using an automated DNA sequencer (such as the Model 373 from Applied Biosystems, Inc.), and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

Unless otherwise indicated, each "nucleotide sequence" set forth herein is presented as a sequence of deoxyribonucleotides (abbreviated A, G, C, and T). However, by "nucleotide sequence" of a nucleic acid molecule or polynucleotide is intended, for a DNA molecule or polynucleotide, a sequence of deoxyribonucleotides, and for an RNA molecule or polynucleotide, the corresponding sequence of ribonucleotides (A, G, C, and U), where each thymidine deoxyribonucleotide (T) in the specified deoxyribonucleotide sequence is replaced by the ribonucleotide uridine (U). For instance, reference to an RNA molecule having the sequence of SEQ ID NO:1 set forth using deoxyribonucleotide abbreviations is intended to indicate an RNA molecule having a sequence in which each deoxyribonucleotide A, G, or C of SEQ ID NO:1 has been replaced by the corresponding ribonucleotide A, G or C, and each deoxyribonucleotide T has been replaced by a ribonucleotide U.

Using the information provided herein, such as the nucleotide sequence in Figure 1, a nucleic acid molecule of the present invention encoding a connective tissue growth factor-3 polypeptide may be obtained using standard cloning and screening procedures, such as those for cloning cDNAs using mRNA as starting material. Illustrative of the invention, the nucleic acid molecule described in Figure 1 (SEQ ID NO:1) was discovered in a cDNA library derived from human osteoblasts. The gene was also identified in cDNA libraries from the following tissues: ovary, testis, heart, lung, skeletal muscle, adrenal medulla, adrenal cortex, thymus, prostate, small intestine, and colon. The determined nucleotide sequence of the connective tissue growth factor-3 cDNA of Figure 1 (SEQ ID NO:1) contains an open reading frame encoding a protein of 250 amino acid residues, with an initiation codon at positions 17-19 of the nucleotide sequence in Figure 1 (SEQ ID NO:1), a predicted leader sequence of about 19 amino acid residues, and a deduced molecular weight of about 26 kDa. The amino acid sequence of the predicted mature connective tissue growth factor-3 is shown in Figure 1 (SEQ ID NO:2) from amino acid residue 20 to residue 250. The connective tissue growth factor-3 protein shown in Figure I (SEQ ID NO:2) is about 44% identical and about 59% similar to human connective tissue growth factor-1 (Figure 2).

As one of ordinary skill would appreciate, due to the possibilities of sequencing errors discussed above, as well as the variability of cleavage sites for leaders in different known proteins, the actual connective tissue growth factor-3 polypeptide encoded by the deposited cDNA comprises about 250 amino acids, but may be anywhere in the range of 235 to 265 amino acids; and the actual leader sequence of this protein is about 19 amino acids, but may be anywhere in the range of about 15 to about 25 amino acids.

As indicated, nucleic acid molecules of the present invention may be in the form of RNA, such as mRNA, or in the form of DNA, including, for instance, cDNA and genomic DNA obtained by cloning or produced synthetically. The DNA may be double-stranded or single-stranded. Single-stranded DNA or RNA may be the coding strand, also known as the sense strand, or it may be the non-coding strand, also referred to as the anti-sense strand.

By "isolated" nucleic acid molecule(s) is intended a nucleic acid molecule, DNA or RNA, which has been removed from its native environment For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically.

Isolated nucleic acid molecules of the present invention include DNA molecules comprising an open reading frame (ORF) with an initiation codon at positions 17-19 of the nucleotide sequence shown in Figure 1 (SEQ ID NO:1); DNA molecules comprising the coding sequence for the mature connective tissue growth factor-3 protein shown in Figure 1 (last 231 amino acids) (SEQ ID NO:2); and DNA molecules which comprise a sequence substantially different from those described above but which, due to the degeneracy of the genetic code, still encode the connective tissue growth factor-3 protein. Of course, the genetic code is well known in the art. Thus, it would be routine for one skilled in the art to generate the degenerate variants described above.

In another aspect, the invention provides isolated nucleic acid molecules encoding the connective tissue growth factor-3 polypeptide having an amino acid sequence encoded by the cDNA clone contained in the plasmid deposited as ATCC Deposit No. 97756 on October 10, 1996. Preferably, this nucleic acid molecule will encode the mature polypeptide encoded by the above-described deposited cDNA clone. The invention further provides an isolated nucleic acid molecule having the nucleotide sequence shown in Figure 1 (SEQ ID NO:1) or the nucleotide sequence of the connective tissue growth factor-3 cDNA contained in the above-described deposited clone, or a nucleic acid molecule having a sequence complementary to one of the above sequences. Such isolated molecules, particularly DNA molecules, are useful as probes for gene mapping, by *in situ* hybridization with chromosomes, and for detecting expression of the connective tissue growth factor-3 gene in human tissue, for instance, by Northern blot analysis.

In another aspect, the invention provides an isolated nucleic acid molecule comprising a polynucleotide which hybridizes under stringent hybridization conditions to a portion of the polynucleotide in a nucleic acid molecule of the invention described above, for instance, the cDNA clone contained in ATCC Deposit 97756) and which encodes a polypeptide that promotes an increase in bone mass. By "stringent hybridization condition" is intended overnight incubation at 42°C in solution comprising: 50% formamide, 5 x SSC (150 mM NaCl, 15 mM trisodium citrate), 50 mM sodium phosphate (pH 7.6), 5x Denhardt's solution, 10% dextran sulfate, and 20 g/ml denatured, sheared salmon sperm DNA, followed by washing the filters in 0.1 x SSC at about 65°C.

By a polynucleotide which hybridizes to a "portion" of a polynucleotide is intended a polynucleotide (either DNA or RNA) hybridizing to at least about 15 nucleotides (nt), and more preferably at least about 20 nt, still more preferably at least about 30 nt, and even more preferably about 30-70 nt of the reference polynucleotide. These are useful as diagnostic probes and primers as discussed above and in more detail below.

Of course, polynucleotides hybridizing to a larger portion of the reference polynucleotide (e.g., the deposited cDNA clone), for instance, a portion 50-750 nt in length, or even to the entire length of the reference polynucleotide; are also useful as probes according to the present invention, as are polynucleotides corresponding to most, if not all, of the nucleotide sequence of the deposited cDNA or the nucleotide sequence as shown in Figure 1 (SEQ ID NO:1). By a portion of a polynucleotide of "at least 20 nt in length," for example, is intended 20 or more contiguous nucleotides from the nucleotide sequence of the reference polynucleotide (e.g., the deposited cDNA or the nucleotide sequence as shown in Figure 1 (SEQ ID NO:1)). As indicated, such portions are useful diagnostically either as a probe according to conventional DNA hybridization techniques or as primers for amplification of a target sequence by the polymerase chain reaction (PCR), as described, for instance, in *Molecular Cloning, A Laboratory Manual*, 2nd. edition, edited by Sambrook, J., Fritsch, E. F. and Maniatis, T., (1989), Cold Spring Harbor Laboratory Press.

Since a connective tissue growth factor-3 cDNA clone has been deposited and its determined nucleotide sequence is provided in Figure 1 (SEQ ID NO:1), generating polynucleotides which hybridize to a portion of the connective tissue growth factor-3 cDNA molecule would be routine to the skilled artisan. For example, restriction endonuclease cleavage or shearing by sonication of the connective tissue growth factor-3 cDNA clone could easily be used to generate DNA portions of various sizes which are polynucleotides that hybridize to a portion of the connective tissue growth factor-3 cDNA molecule. Alternatively, the hybridizing polynucleotides of the present invention could be generated synthetically according to known techniques. Of course, a polynucleotide which hybridizes only to a poly A sequence (such as the 3' terminal poly(A) tract of the connective tissue growth factor-3 cDNA shown in Figure 1 (SEQ ID NO:1)), or to a complementary stretch of T (or U) resides, would not be included in a polynucleotide of the invention used to hybridize to a portion of a nucleic acid of the invention, since such a polynucleotide would hybridize to any nucleic acid molecule containing a poly (A) stretch or the complement thereof (e.g., practically any double-stranded cDNA clone).

As indicated, nucleic acid molecules of the present invention which encode a connective tissue growth factor-3 polypeptide may include, but are not limited to those encoding the amino acid sequence of the mature polypeptide, by itself; the coding sequence for the mature polypeptide and additional sequences, such as those encoding the about 19 amino acid leader or secretory sequence, such as a pre-, or pro- or prepro- protein sequence; the coding sequence of the mature polypeptide, with or without the aforementioned additional coding sequences, together with additional, non-coding sequences, including for example, non-coding 5' and 3' sequences, ribosome binding and stability of mRNA; an additional coding sequence which codes for additional amino acids, such as those which provide additional functionalities. Thus, the sequence encoding the polypeptide may be fused to a marker sequence, such as a sequence encoding a peptide which facilitates purification of the fused polypeptide. In certain preferred embodiments of this aspect of the invention, the marker amino acid sequence is a hexa-histidine peptide, such as the tag provided in a pQE vector (Qiagen, Inc.), among others, many of which are commercially available. As described in Gentz *et al., Proc. Natl. Acad Sci. USA 86*:821-824 (1989), for instance, hexa-histidine provides for convenient purification of the fusion protein. The "HA" tag is another peptide useful for purification which corresponds to an epitope derived from the influenza hemagglutinin protein, which has been described by Wilson *et al., Cell 3*7: 767 (1984). As discussed below, other such fusion proteins include the connective tissue growth factor-3 fused to Fc at the N- or C-terminus.

The present invention further describes variants of the nucleic acid molecules of the present invention, which encode analogs, or derivatives of the connective tissue growth factor-3 protein. Variants may occur naturally, such as a natural allelic variant. By an "allelic variant" is intended one of several alternate forms of a gene occupying a given locus on a chromosome of an organism. *Genes II,* Lewin, B., ed., John Wiley & Sons, New York (1985). Non-naturally occurring variants may be produced using art-known mutagenesis techniques.

Such variants include those produced by nucleotide substitutions, deletions, or additions. The substitutions, deletions or additions may involve one or more nucleotides. The variants may be altered in coding regions, non-coding regions, or both. Alterations in the coding regions may produce conservative or non-conservative amino acid substitutions, deletions, or additions. Especially preferred among these are silent substitutions, additions, and, which do not alter the properties and activities of the connective tissue growth factor-3 protein or portions thereof. Also especially preferred in this regard are conservative substitutions. Most highly preferred are nucleic acid molecules encoding the mature protein having the amino acid sequence shown in Figure 1 (SEQ ID NO:2) or the mature connective tissue growth factor-3 amino acid sequence encoded by the deposited cDNA clone.

Further embodiments of the invention include isolated nucleic acid molecules comprising a polynucleotide having a nucleotide sequence at least 95%, 96%, 97%, 98%, or 99% identical to (a) a nucleotide sequence encoding the full-length connective tissue growth factor-3 polypeptide having the complete amino acid sequence in Figure 1 (SEQ ID NO:2), including the predicted leader sequence; (b) a nucleotide sequence encoding the mature connective tissue growth factor-3 polypeptide (full-length polypeptide with the leader removed) having the amino acid sequence at positions 20 to 250 in Figure 1 (SEQ ID NO:2); (c) a nucleotide sequence encoding the full-length connective tissue growth factor-3 polypeptide having the complete amino acid sequence including the leader encoded by the cDNA clone contained in ATCC Deposit No. 97756; (d) a nucleotide sequence encoding the mature connective tissue growth factor-3 polypeptide having the amino acid sequence encoded by the cDNA clone contained in ATCC Deposit No. 97756; or (e) a nucleotide sequence complementary to any of the nucleotide sequences in (a), (b), (c), or (d).

By a polynucleotide having a nucleotide sequence at least, for example, 95% "identical" to a reference nucleotide sequence encoding a connective tissue growth factor-3 polypeptide is intended that the nucleotide sequence of the polynucleotide is identical to the reference sequence except that the polynucleotide sequence may include up to five point mutations per each 100 nucleotides of the reference nucleotide sequence encoding the connective tissue growth factor-3 polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 95%, 96%, 97%, 98% or 99% identical to, for instance, the nucleotide sequence shown in Figure 1 or to the nucleotides sequence of the deposited cDNA clone can be determined conventionally using known computer programs such as the Bestfit program (Wisconsin Sequence Analysis Package, Version 8 for Unix, Genetics Computer Group, University Research Park, 575 Science Drive; Madison, WI 53711). Bestfit uses the local homology algorithm of Smith and Waterman, Advances in Applied Mathematics 2: 482-489 (1981), to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

The present application is directed to nucleic acid molecules at least 95%, 96%, 97%, 98%, or 99% identical to the nucleic acid sequence shown in Figure 1 (SEQ ID NO:1) or to the nucleic acid sequence of the deposited cDNA, irrespective of whether they encode a polypeptide having connective tissue growth factor-3 activity. This is because even where a particular nucleic acid molecule does not encode a polypeptide having connective tissue growth factor-3 activity, one of skill in the art would still know how to use the nucleic acid molecule, for instance, as a hybridization probe or a polymerase chain reaction (PCR) primer. Uses of the nucleic acid molecules of the present invention that do not encode a polypeptide having connective tissue growth factor-3 activity include, inter alia, (1) isolating the connective tissue growth factor-3 gene or allelic variants thereof in a cDNA library; (2) in situ hybridization (e.g., "FISH") to metaphase chromosomal spreads to provide precise chromosomal location of the connective tissue growth factor-3 gene, as described in Verma *et al*., Human Chromosomes: A Manual of Basic Techniques, Pergamon Press, New York (1988); and Northern Blot analysis for detecting connective tissue growth factor-3 mRNA expression in specific tissues.

Preferred, however, are nucleic acid molecules having sequences at least 95%, 96%, 97%, 98%, or 99% identical to the nucleic acid sequence shown in Figure 1 (SEQ ID NO:1) or to the nucleic acid sequence of the deposited cDNA which do, in fact, encode a polypeptide having connective tissue growth factor-3 protein activity. By "a polypeptide having connective tissue growth factor-3 activity" is intended polypeptides exhibiting activity similar, but not necessarily identical, to an activity of the connective tissue growth factor-3 protein of the invention (either the full-length protein or, preferably, the mature protein), as measured in a particular biological assay. It is believed that connective tissue growth factor-3 will have chemotactic and mitogenic activity for connective tissue cells, similar to platelet-derived growth factor (PDGF). Assays to test these activities are described in DiCorleto, P.E., *Exp. Cell. Res. 153*:167-172 (1984). In addition, it is believed that connective tissue growth factor-3 activity will include an increased synthesis of extracellular matrix/connective tissue components, such as, e..g, collagen fibronectin, PA1-1, syndecan, and elastin. This activity can be tested by Northern and Western blot or ELISA analyses after treatment of cultured cells with CTGF-3 protein.

Thus, "a polypeptide having connective tissue growth factor-3 protein activity" includes polypeptides that exhibit connective tissue growth factor-3 activity, in the above-described assay. Although the degree of activity need not be identical to that of the connective tissue growth factor-3 protein, preferably, "a polypeptide having connective tissue growth factor-3 protein activity" will exhibit substantially similar activity as compared to the connective tissue growth factor-3 protein (i.e., the candidate polypeptide will exhibit greater activity or not more than about twenty-fold less and, preferably, not more than about ten-fold less activity relative to the reference connective tissue growth factor-3 protein).

Of course, due to the degeneracy of the genetic code, one of ordinary skill in the art will immediately recognize that a large number of the nucleic acid molecules having a sequence at least 95%, 96%, 97%, 98%, or 99% identical to the nucleic acid sequence of the deposited cDNA or the nucleic acid sequence shown in Figure 1 (SEQ ID NO:1) will encode a polypeptide "having connective tissue growth factor-3 protein activity." In fact, since degenerate variants of these nucleotide sequences all encode the same polypeptide, this will be clear to the skilled artisan even without performing the above described comparison assay. It will be further recognized in the art that, for such nucleic acid molecules that are not degenerate variants, a reasonable number will also encode a polypeptide having connective tissue growth factor-3 protein activity. This is because the skilled artisan is fully aware of amino acid substitutions that are either less likely or not likely to significantly effect protein function (e.g., replacing one aliphatic amino acid with a second aliphatic amino acid).

For example, guidance concerning how to make phenotypically silent amino acid substitutions is provided in Bowie, J. U. *et al*., "Deciphering the Message in Protein Sequences: Tolerance to Amino Acid Substitutions," *Science 247:*1306-1310 (1990), wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selections or screens to identify sequences that maintain functionality. As the authors state, these studies have revealed that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described in Bowie, J.U. *et al*., *supra,* and the references cited therein.

### Vectors and Host Cells

The present invention also relates to vectors that include the isolated DNA molecules of the present invention, host cells that are genetically engineered with the recombinant vectors, and the production of connective tissue growth factor-3 polypeptides or fragments thereof by recombinant techniques.

Recombinant constructs may be introduced into host cells using well known techniques such as infection, transduction, transfection, transvection, electroporation, and transformation. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells.

Preferred are vectors comprising cis-acting control regions to the polynucleotide of interest. Appropriate trans-acting factors may be supplied by the host, supplied by a complementing vector or supplied by the vector itself upon introduction into the host.

In certain preferred embodiments in this regard, the vectors provide for specific expression, which may be inducible and/or cell type-specific. Particularly preferred among such vectors are those inducible by environmental factors that are easy to manipulate, such as temperature and nutrient additives.

Expression vectors useful in the present invention include chromosomal-, episomal- and virus-derived vectors, e.g., vectors derived from bacterial plasmids, bacteriophage, yeast episomes, yeast chromosomal elements, viruses such as baculoviruses, papova viruses, vaccinia viruses, adenoviruses, fowl pox viruses, pseudorabies viruses and retroviruses, and vectors derived from combinations thereof, such as cosmids and phagemids.

The DNA insert should be operatively linked to an appropriate promoter, such as the phage lambda PL promoter, the *E. coli lac, trp*, and *tac* promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (UAA, UGA or UAG) appropriately positioned at the end of the polypeptide to be translated.

As indicated, the expression vectors will preferably include at least one selectable marker. Such markers include dihydrofolate reductase or neomycin resistance for eukaryotic cell culture and tetracycline or ampicillin resistance genes for culturing in *E. coli* and other bacteria. Representative examples of appropriate hosts include, but arc not limited to, bacterial cells, such as *E. coli, Streptomyces* and *Salmonella typhimurium* cells; fungal cells, such as yeast cells; insect cells such as *Drosophila* S2 and *Spodoptera* Sf9 cells; animal cells such as CHO, COS, and Bowes melanoma cells; and plant cells. Appropriate culture mediums and conditions for the above-described host cells are known in the art.

Among vectors preferred for use in bacteria include pQE70, pQE60, and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Among known bacterial promoters suitable for use in the present invention include the *E. coli lac*I and *lac*Z promoters, the T3 and T7 promoters, the *gpt* promoter, the lambda PR and PL promoters and the *trp* promoter. Suitable eukaryotic promoters include the CMV immediate early promoter, the HSV thymidine kinase promoter, the early and late SV40 promoters, the promoters of retroviral LTRs, such as those of the Rous sarcoma virus (RSV), and metallothionein promoters, such as the mouse metallothionein-I promoter.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis *et al., Basic Methods In Molecular Biology* (1986).

Transcription of the DNA encoding the polypeptides of the present invention by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretion signals may be incorporated into the expressed polypeptide. The signals may be endogenous to the polypeptide or they may be heterologous signals.

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected, and purified in the advantageous manner described. This is the case when the Fc portion proves to be a hindrance to use in therapy and diagnosis, for example, when the fusion protein is to be used as antigen for immunizations. In drug discovery, for example, human proteins, such as, hIL5-, have been fused with Fc portions for the purpose of high-throughput screening assays to identify antagonists of hIL-5. See, D. Bennett *et al., Journal of Molecular Recognition 8:*52-58 (1995) and K. Johanson *et al., The Journal of Biological Chemistry 270:*9459-9471 (1995).

The connective tissue growth factor-3 protein can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

### Connective Tissue Growth Factor-3 Polypeptides and Fragments

The invention further provides an isolated connective tissue growth factor-3 polypeptide having the amino acid sequence encoded by the deposited cDNA, or the amino acid sequence in Figure 1 (SEQ ID NO:2), or a peptide or polypeptide comprising a portion of the above polypeptides. The terms "peptide" and "oligopeptide" are considered synonymous (as is commonly recognized) and each term can be used interchangeably as the context requires to indicate a chain of at least two amino acids coupled by peptidyl linkages. The word "polypeptide" is used herein for chains containing more than ten amino acid residues. All oligopeptide and polypeptide formulas or sequences herein are written from left to right and in the direction from amino terminus to carboxy terminus.

It will be recognized in the art that some amino acid sequences of the connective tissue growth factor-3 polypeptide can be varied without significant effect of the structure or function of the protein. If such differences in sequence are contemplated, it should be remembered that there will be critical areas on the protein which determine activity. In general, it is possible to replace residues which form the tertiary structure, provided that residues performing a similar function are used. In other instances, the type of residue may be completely unimportant if the alteration occurs at a non-critical region of the protein.

Thus, the invention further describes variations of the connective tissue growth factor-3 polypeptide that show substantial connective tissue growth factor-3 polypeptide activity or that include regions of connective tissue growth factor-3 protein, such as the protein portions discussed below. Such mutants include deletions, insertions, inversions, repeats, and type substitutions (for example, substituting one hydrophilic residue for another, but not strongly hydrophilic for strongly hydrophobic as a rule). Small changes or such "neutral" amino acid substitutions will generally have little effect on activity.

Typically seen as conservative substitutions are the replacements, one for another, among the aliphatic amino acids Ala, Val, Leu, and Ile; interchange of the hydroxyl residues Ser and Thr, exchange of the acidic residues Asp and Glu, substitution between the amide residues Asn and Gln, exchange of the basic residues Lys and Arg, and replacements among the aromatic residues Phe and Tyr.

As indicated in detail above, further guidance concerning which amino acid changes are likely to be phenotypically silent (i.e., are not likely to have a significant deleterious effect on a function) can be found in Bowie, J.U., *et al*., "Deciphering the Message in Protein Sequences: Tolerance to Amino Acid Substitutions," *Science 247:*1306-1310 (1990).

The polypeptides of the present invention are preferably provided in an isolated form, and preferably are substantially purified. A recombinantly produced version of the connective tissue growth factor-3 polypeptide can be substantially purified by the one-step method described in Smith and Johnson, *Gene 67:*31-40 (1988).

The polypeptides of the present invention include the polypeptide encoded by the deposited cDNA including the leader, the mature polypeptide encoded by the deposited the cDNA minus the leader (i.e., the mature protein), the polypeptide of Figure 1 (SEQ ID NO:2) including the leader, the polypeptide of Figure 1 (SEQ ID NO:2) minus the leader, as well as polypeptides which have at least 95% similarity, and still more preferably at least 96%, 97%, 98%, or 99% similarity to those described above. Further polypeptides of the present invention include polypeptides at least 95% identical, still more preferably at least 96%, 97%, 98%, or 99% identical to the polypeptide encoded by the deposited cDNA, to the polypeptide of Figure 1 (SEQ ID NO:2).

By "% similarity" for two polypeptides is intended a similarity score produced by comparing the amino acid sequences of the two polypeptides using the Bestfit program (Wisconsin Sequence Analysis Package, Version 8 for Unix, Genetics Computer Group, University Research Park, 575 Science Drive, Madison, WI 53711) and the default settings for determining similarity. Bestfit uses the local homology algorithm of Smith and Waterman *(Advances in Applied Mathematics 2*: 482-489, 1981) to find the best segment of similarity between two sequences.

By a polypeptide having an amino acid sequence at least, for example, 95% "identical" to a reference amino acid sequence of a connective tissue growth factor-3 polypeptide is intended that the amino acid sequence of the polypeptide is identical to the reference sequence except that the polypeptide sequence may include up to five amino acid alterations per each 100 amino acids of the reference amino acid of the connective tissue growth factor-3 polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequence shown in Figure 1 (SEQ ID NO:2) or to the amino acid sequence encoded by deposited cDNA clone can be determined conventionally using known computer programs such the Bestfit program (Wisconsin Sequence Analysis Package, Version 8 for Unix, Genetics Computer Group, University Research Park, 575 Science Drive, Madison, WI 53711. When using Bestfit or any other sequence alignment program to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

The polypeptide of the present invention could be used as a molecular weight marker on SDS-PAGE gels or on molecular sieve gel filtration columns using methods well known to those of skill in the art.

As described in detail below, the polypeptides of the present invention can also be used to raise polyclonal and monoclonal antibodies, which are useful in assays for detecting connective tissue growth factor-3 protein expression as described below or as agonists and antagonists capable of enhancing or inhibiting connective tissue growth factor-3 protein function. Further, such polypeptides can be used in the yeast two-hybrid system to "capture" connective tissue growth factor-3 protein binding proteins which are also candidate agonist and antagonist according to the present invention. The yeast two hybrid system is described in Fields and Song, *Nature 340:*245-246 (1989).

Antigenic epitope-bearing peptides and polypeptides of the invention are therefore useful to raise antibodies, including monoclonal antibodies, that bind specifically to a polypeptide of the invention. Thus, a high proportion of hybridomas, obtained by fusion of spleen cells from donors immunized with an antigen epitope-bearing peptide, generally secrete antibody, reactive with the native protein. Sutcliffe *et al., supra*, at 663. The antibodies raised by antigenic epitope-bearing peptides or polypeptides are useful to detect the mimicked protein, and antibodies to different peptides may be used for tracking the fate of various regions of a protein precursor which undergo post-translational processing. The peptides and anti-peptide antibodies may be used in a variety of qualitative or quantitative assays for the mimicked protein, for instance, in competition assays since it has been shown that even short peptides (e.g., about 9 amino acids) can bind and displace the larger peptides in immunoprecipitation assays. See, for instance, Wilson *et al., Cell 37*:767-778 (1984) at 777. The anti-peptide antibodies of the invention also are useful for purification of the mimicked protein, for instance, by adsorption chromatography using methods well known in the art.

Non-limiting examples of antigenic polypeptides or peptides that can be used to generate connective tissue growth factor-3-specific antibodies include: a polypeptide comprising amino acid residues from about 55 to about 68 in Figure 1 (SEQ ID NO:2); a polypeptide comprising amino acid residues from about 94 to about 128 in Figure 1 (SEQ ID NO:2); a polypeptide comprising amino acid residues from about 134 to about 158 in Figure 1 (SEQ ID NO:2); and a polypeptide comprising amino acid residues from about 215 to about 249 in Figure 1 (SEQ ID NO: 2). As indicated above, the inventors have determined that the above polypeptide fragments are antigenic regions of the connective tissue growth factor-3 protein.

The epitope-bearing peptides and polypeptides of the invention may be produced by any conventional means for making peptides or polypeptides including recombinant means using nucleic acid molecules of the invention. For instance, a short epitope-bearing amino acid sequence may be fused to a larger polypeptide which acts as a carrier during recombinant production and purification, as well as during immunization to produce anti-peptide antibodies. Epitope-bearing peptides also may be synthesized using known methods of chemical synthesis. For instance, Houghten has described a simple method for synthesis of large numbers of peptides, such as 10-20 mg of 248 different 13 residue peptides representing single amino acid variants of a segment of the HA1 polypeptide which were prepared and characterized (by ELISA-type binding studies) in less than four weeks. Houghten, R. A., "General Method for the Rapid Solid-Phase Synthesis of Large Numbers of Peptides: Specificity of Antigen-Antibody Interaction at the Level of Individual Amino Acids," *Proc. Natl. Acad. Sci. USA 82*:5131-5135 (1985). This "Simultaneous Multiple Peptide Synthesis (SMPS)" process is further described in U.S. Patent No. 4,631,211 to Houghten *et al.* (1986). In this procedure, the individual resins for the solid-phase synthesis of various peptides are contained in separate solvent-permeable packets, enabling the optimal use of the many identical repetitive steps involved in solid-phase methods. A completely manual procedure allows 500-1000 or more syntheses to be conducted simultaneously. Houghten *et al., supra,* at 5134.

Epitope-bearing peptides and polypeptides of the invention are used to induce antibodies according to methods well known in the art. See, for instance, Sutcliffe *et al*., *supra*; Wilson *et al., supra*; Chow, M. *et al., Proc. Natl. Acad. Sci. USA 82:*910-914 (1985); and Bittle, F. J. *et al., J Gen. Virol. 66*:2347-2354 (1985). Generally, animals may be immunized with free peptide; however, anti-peptide antibody titer may be boosted by coupling of the peptide to a macromolecular carrier, such as keyhole limpet hemocyanin (KLH) or tetanus toxoid. For instance, peptides containing cysteine may be coupled to carrier using a linker such as m-maleimidobenzoyl-N-hydroxysuccinimide ester (MBS), while other peptides may be coupled to carrier using a more general linking agent such as glutaraldehyde. Animals such as rabbits, rats, and mice are immunized with either free or carrier-coupled peptides, for instance, by intraperitoneal and/or intradermal injection of emulsions containing about 100 g peptide or carrier protein and Freund's adjuvant. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

Immunogenic epitope-bearing peptides of the invention, i.e., those parts of a protein that elicit an antibody response when the whole protein is the immunogen, are identified according to methods known in the art. For instance, Geysen *et al*., *supra*, disclose a procedure for rapid concurrent synthesis on solid supports of hundreds of peptides of sufficient purity to react in an enzyme-linked immunosorbent assay. Interaction of synthesized peptides with antibodies is then easily detected without removing them from the support. In this manner, a peptide bearing an immunogenic epitope of a desired protein may be identified routinely by one of ordinary skill in the art. For instance, the immunologically important epitope in the coat protein of foot-and-mouth disease virus was located by Geysen *et al*. with a resolution of seven amino acids by synthesis of an overlapping set of all 208 possible hexapeptides covering the entire 213 amino acid sequence of the protein. Then, a complete replacement set of peptides in which all 20 amino acids were substituted in turn at every position within the epitope were synthesized, and the particular amino acids conferring specificity for the reaction with antibody were determined. Thus, peptide analogs of the epitope-bearing peptides of the invention can be made routinely by this method. U.S. Patent No. 4,708,781 to Geysen (1987) further describes this method of identifying a peptide bearing an immunogenic epitope of a desired protein.

Further still, U.S. Patent No. 5,194,392 to Geysen (1990) describes a general method of detecting or determining the sequence of monomers (amino acids or other compounds) which is a topological equivalent of the epitope (i.e., a "mimotope"), which is complementary to a particular paratope (antigen binding site) of an antibody of interest. More generally, U.S. Patent No. 4,433,092 to Geysen (1989) describes a method of detecting or determining a sequence of monomers which is a topographical equivalent of a ligand which is complementary to the ligand binding site of a particular receptor of interest. Similarly, U.S. Patent No. 5,480,971 to Houghten, R. A. *et al*. (1996) entitled "Peralkylated Oligopeptide Mixtures" discloses linear C₁-C₇-alkyl peralkylated oligopeptides and sets and libraries of such peptides, as well as methods for using such oligopeptide sets and libraries for determining the sequence of a peralkylated oligopeptide that preferentially binds to an acceptor molecule of interest. Thus, non-peptide analogs of the epitope-bearing peptides of the invention also can be made routinely by these methods.

As one of skill in the art will appreciate, connective tissue growth factor-3 polypeptides of the present invention and the epitope-bearing fragments thereof described above can be combined with parts of the constant domain of immunoglobulins (IgG), resulting in chimeric polypeptides. These fusion proteins facilitate purification and show an increased half-life *in vivo.* This has been shown, e.g., for chimeric proteins consisting of the first two domains of the human CD4-polypeptide and various domains of the constant regions of the heavy or light chains of mammalian immunoglobulins (EPA 394,827; Traunecker *et al., Nature 331*:84- 86 (1988)). Fusion proteins that have a disulfide-linked dimeric structure due to the IgG part can also be more efficient in binding and neutralizing other molecules than the monomeric connective tissue growth factor-3 protein or protein fragment alone (Fountoulakis *et al., J Biochem 270*:3958-3964 (1995)).

### Diagnosis and Prognosis of Connective Tissue Related Disorders

It is believed that certain tissues in mammals with various connective-tissue related disorders express significantly altered levels of the connective tissue growth factor-3 protein and mRNA encoding the connective tissue growth factor-3 protein, when compared to a corresponding "standard" mammal, i.e., a mammal of the same species not having the disorder. By "connective tissue related disorders" is intended any disease or condition that is caused by, associated with, or characterized by an over or under growth of connective tissue cells. Some non-limiting examples of such disorders include cancer, arthritis, fibrosis, atherosclerosis, and, in particular, osteoporosis.

For example, it is believed that enhanced levels of the connective tissue growth factor-3 protein can be detected in certain body fluids (e.g., sera, plasma, urine, and spinal fluid) or tissues from mammals with cancer, fibrosis, arthritis, or atherosclerosis when compared to sera from mammals of the same species not having these diseases. Thus, the invention describes a diagnostic method useful during diagnosis of connective-tissue related disorders, such as cancer, fibrosis, arthritis, or artherosclerosis, which involves assaying the expression level of the gene encoding the connective tissue growth factor-3 protein in mammalian cells or body fluid and comparing the gene expression level with a standard connective tissue growth factor-3 gene expression level, whereby an increase in the gene expression level over the standard is indicative of these diseases.

Where a diagnosis of any of these diseases has already been made according to conventional methods, the present invention is also useful as a prognostic indicator, whereby patients exhibiting enhanced connective tissue growth factor-3 gene expression will experience a worse clinical outcome relative to patients expressing the gene at a lower level.

It is also believed that decreased levels of the connective tissue growth factor-3 protein can be detected in certain body fluids (e.g., sera, plasma, urine, and spinal fluid) or tissues from mammals with certain connective-tissue related disorders, such as osteoporosis, when compared to sera from mammals of the same species not having the disease. Thus, the invention provides a diagnostic method useful during diagnosis of connective-tissue related disorders, preferably osteoporosis, which involves assaying the expression level of the gene encoding the connective tissue growth factor-3 protein in mammalian cells or body fluid and comparing the gene expression level with a standard connective tissue growth factor-3 gene expression level, whereby a decrease in the gene expression level over the standard is indicative of the disease.

By "assaying the expression level of the gene encoding the connective tissue growth factor-3 protein" is intended qualitatively or quantitatively measuring or estimating the level of the connective tissue growth factor-3 protein or the level of the mRNA encoding the connective tissue growth factor-3 protein in a first biological sample either directly (e.g., by determining or estimating absolute protein level or mRNA level) or relatively (e.g., by comparing to the connective tissue growth factor-3 protein level or mRNA level in a second biological sample).

Preferably, the connective tissue growth factor-3 protein level or mRNA level in the first biological sample is measured or estimated and compared to a standard connective tissue growth factor-3 protein level or mRNA level, the standard being taken from a second biological sample obtained from an individual not having the connective-tissue related disorder. As will be appreciated in the art, once a standard connective tissue growth factor-3 protein level or mRNA level is known, it can be used repeatedly as a standard for comparison.

By "biological sample" is intended any biological sample obtained from an individual, cell line, tissue culture, or other source which contains connective tissue growth factor-3 protein or mRNA. Biological samples include mammalian body fluids (such as sera, plasma, urine, synovial fluid and spinal fluid) which contain secreted mature connective tissue growth factor-3 protein, and ovarian, testicular, prostate, heart, placenta, pancreas liver, spleen, lung, breast and umbilical tissue. Methods for obtaining tissue biopsies and body fluids from mammals are well known in the art. Where the biological sample is to include mRNA, a tissue biopsy is the preferred source.

The present invention may be useful for detecting osteoporosis. Preferred mammals include monkeys, apes, cats, dogs, cows, pigs, horses, rabbits and humans. Particularly preferred are humans.

Total cellular RNA can be isolated from a biological sample using any suitable technique such as the single-step guanidinium-thiocyanate-phenolchloroform method described in Chomczynski and Sacchi, *Anal. Biochem. 162:*156-159 (1987). Levels of mRNA encoding the connective tissue growth factor-3 protein are then assayed using any appropriate method. These include Northern blot analysis, S1 nuclease mapping, the polymerase chain reaction (PCR), reverse transcription in combination with the polymerase chain reaction (RT-PCR), and reverse transcription in combination with the ligase chain reaction (RT-LCR).

Northern blot analysis can be performed as described in Harada *et al., Cell 63:*303-312 (1990). Briefly, total RNA is prepared from a biological sample as described above. For the Northern blot, the RNA is denatured in an appropriate buffer (such as glyoxal/dimethyl sulfoxide/sodium phosphate buffer), subjected to agarose gel electrophoresis, and transferred onto a nitrocellulose filter. After the RNAs have been linked to the filter by a UV linker, the filter is prehybridized in a solution containing formamide, SSC, Denhardt's solution, denatured salmon sperm, SDS, and sodium phosphate buffer. Connective tissue growth factor-3 protein cDNA labeled according to any appropriate method (such as the ³²P-multiprimed DNA labeling system (Amersham)) is used as probe. After hybridization overnight, the filter is washed and exposed to x-ray film, cDNA for use as a probe according to the present invention is described in the sections above, and will preferably be at least 15 bp in length.

S1 mapping can be performed as described in Fujita *et al*., *Cell 49:*357-367 (1987). To prepare probe DNA for use in S1 mapping, the sense strand of the above-described cDNA is used as a template to synthesize labeled antisense DNA. The antisense DNA can then be digested using an appropriate restriction endonuclease to generate further DNA probes of a desired length. Such antisense probes are useful for visualizing protected bands corresponding to the target mRNA (i.e., mRNA encoding the connective tissue growth factor-3 protein). Northern blot analysis can be performed as described above.

Preferably, levels of mRNA encoding the connective tissue growth factor-3 protein are assayed using the RT-PCR method described in Makino *et al., Technique 2:*295-301 (1990). By this method, the radioactivities of the "amplicons" in the polyacrylamide gel bands are linearly related to the initial concentration of the target mRNA. Briefly, this method involves adding total RNA isolated from a biological sample in a reaction mixture containing a RT primer and appropriate buffer. After incubating for primer annealing, the mixture can be supplemented with a RT buffer, dNTPs, DTT, RNase inhibitor and reverse transcriptase. After incubation to achieve reverse transcription of the RNA, the RT products are then subject to PCR using labeled primers. Alternatively, rather than labeling the primers, a labeled dNTP can be included in the PCR reaction mixture. PCR amplification can be performed in a DNA thermal cycler according to conventional techniques. After a suitable number of rounds to achieve amplification, the PCR reaction mixture is electrophoresed on a polyacrylamide gel. After drying the gel, the radioactivity of the appropriate bands (corresponding to the mRNA encoding the connective tissue growth factor-3 protein)) is quantified using an imaging analyzer. RT and PCR reaction ingredients and conditions, reagent and gel concentrations, and labeling methods are well known in the art. Variations on the RT-PCR method will be apparent to the skilled artisan.

Any set of oligonucleotide primers that will amplify reverse transcribed target mRNA can be used and can be designed as described in the sections above.

Assaying connective tissue growth factor-3 protein levels in a biological sample can occur using any art-known method. Preferred for assaying connective tissue growth factor-3 protein levels in a biological sample are antibody-based techniques. For example, connective tissue growth factor-3 protein expression in tissues can be studied with classical immunohistological methods. In these, the specific recognition is provided by the primary antibody (polyclonal or monoclonal) but the secondary detection system can utilize fluorescent, enzyme, or other conjugated secondary antibodies. As a result, an immunohistological staining of tissue section for pathological examination is obtained. Tissues can also be extracted, e.g., with urea and neutral detergent, for the liberation of connective tissue growth factor-3 protein for Western-blot or dot/slot assay (Jalkanen, M., *et al., J. Cell. Biol. 101:*976-985 (1985); Jalkanen, M., *et al., J Cell. Biol. 105:*3087-3096 (1987)). In this technique, which is based on the use of cationic solid phases, quantitation of connective tissue growth factor-3 protein can be accomplished using isolated connective tissue growth factor-3 protein as a standard. This technique can also be applied to body fluids. With these samples, a molar concentration of connective tissue growth factor-3 protein will aid to set standard values of connective tissue growth factor-3 protein content for different body fluids, like serum, plasma, urine, spinal fluid, etc. The normal appearance of connective tissue growth factor-3 protein amounts can then be set using values from healthy individuals, which can be compared to those obtained from a test subject.

Other antibody-based methods useful for detecting connective tissue growth factor-3 protein gene expression include immunoassays, such as the enzyme linked immunosorbent assay (ELISA) and the radioimmunoassay (RIA). For example, a connective tissue growth factor-3 protein-specific monoclonal antibody can be used both as an immunoabsorbent and as an enzyme-labeled probe to detect and quantify the connective tissue growth factor-3 protein. The amount of connective tissue growth factor-3 protein present in the sample can be calculated by reference to the amount present in a standard preparation using a linear regression computer algorithm. Such an ELISA for detecting a tumor antigen is described in Iacobelli *et al., Breast Cancer Research and Treatment 11:*19-30 (1988). In another ELISA assay, two distinct specific monoclonal antibodies can be used to detect connective tissue growth factor-3 protein in a body fluid. In this assay, one of the antibodies is used as the immunoabsorbent and the other as the enzyme-labeled probe.

The above techniques may be conducted essentially as a "one-step" or "two-step" assay. The "one-step" assay involves contacting connective tissue growth factor-3 protein with immobilized antibody and, without washing, contacting the mixture with the labeled antibody. The "two-step" assay involves washing before contacting the mixture with the labeled antibody. Other conventional methods may also be employed as suitable. It is usually desirable to immobilize one component of the assay system on a support, thereby allowing other components of the system to be brought into contact with the component and readily removed from the sample.

Suitable enzyme labels include, for example, those from the oxidase group, which catalyze the production of hydrogen peroxide by reacting with substrate. Glucose oxidase is particularly preferred as it has good stability and its substrate (glucose) is readily available. Activity of an oxidase label may be assayed by measuring the concentration of hydrogen peroxide formed by the enzyme-labelled antibody/substrate reaction. Besides enzymes, other suitable labels include radioisotopes, such as iodine (¹²⁵I, ¹³¹I), carbon (¹⁴C), sulphur (³⁵S), tritium (³H), indium (¹¹²In), and technetium (^{99m}Tc), and fluorescent labels, such as fluorescein and rhodamine, and biotin.

In addition to assaying connective tissue growth factor-3 protein levels in a biological sample obtained from an individual, connective tissue growth factor-3 protein can also be detected *in vivo* by imaging. Antibody labels or markers for *in vivo* imaging of connective tissue growth factor-3 protein include those detectable by X-radiography, NMR, or ESR. For X-radiography, suitable labels include radioisotopes such as barium or cesium, which emit detectable radiation, but are not overtly harmful to the subject. Suitable markers for NMR and ESR include those with a detectable characteristic spin, such as deuterium, which may be incorporated into the antibody by labeling of nutrients for the relevant hybridoma.

A connective tissue growth factor-3 protein-specific antibody or antibody fragment which has been labeled with an appropriate detectable imaging moiety, such as a radioisotope (for example, ¹³¹I, ¹¹²In, ^{99m}Tc), a radio-opaque substance, or a material detectable by nuclear magnetic resonance, is introduced (for example, parenterally, subcutaneously or intraperitoneally) into the mammal to be examined for cancer. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ^{99m}Tc. The labeled antibody or antibody fragment will then preferentially accumulate at the location of cells which contain connective tissue growth factor-3 protein. *In vivo* tumor imaging is described in S.W. Burchiel *et al*., "Immunopharmacokinetics of Radiolabelled Antibodies and Their Fragments" (Chapter 13 in *Tumor Imaging: The Radiochemical Detection of Cancer,* eds., S.W. Burchiel and B.A. Rhodes, Masson Publishing, Inc. (1982)).

Connective tissue growth factor-3-protein specific antibodies for use in the present invention can be raised against the intact connective tissue growth factor-3 protein or an antigenic polypeptide fragment thereof, which may be presented together with a carrier protein, such as an albumin, to an animal system (such as rabbit or mouse) or, if it is long enough (at least about 25 amino acids), without a carrier.

As used herein, the term "antibody" (Ab) or "monoclonal antibody" (Mab) is meant to include intact molecules as well as antibody fragments (such as, for example, Fab and F(ab')₂ fragments) which are capable of specifically binding to connective tissue growth factor-3 protein. Fab and F(ab')₂ fragments lack the Fc fragment of intact antibody, clear more rapidly from the circulation, and may have less non-specific tissue binding of an intact antibody (Wahl *et al., J Nucl. Med. 24*:316-325 (1983)). Thus, these fragments are preferred.

The antibodies of the present invention may be prepared by any of a variety of methods. For example, cells expressing the connective tissue growth factor-3 protein or an antigenic fragment thereof can be administered to an animal in order to induce the production of sera containing polyclonal antibodies. In a preferred method, a preparation of connective tissue growth factor-3 protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or connective tissue growth factor-3 protein binding fragments thereof). Such monoclonal antibodies can be prepared using hybridoma technology (Kohler *et al., Nature 256*:495 (1975); Kohler *et al., Eur. J. Immunol. 6*:511 (1976); Kohler *et al., Eur. J. Immunol. 6*:292 (1976); Hammerling *et al*., In: *Monoclonal Antibodies and T-Cell Hybridomas*, Elsevier, N.Y., pp. 563-681 (1981)). In general, such procedures involve immunizing an animal (preferably a mouse) with a connective tissue growth factor-3 protein antigen or, more preferably, with a connective tissue growth factor-3 protein-expressing cell. Suitable cells can be recognized by their capacity to bind anti-connective tissue growth factor-3 protein antibody. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 g/ml of streptomycin. The splenocytes of such mice are extracted and fused with a suitable myeloma cell line. Any suitable myeloma cell line may be employed in accordance with the present invention; however, it is preferable to employ the parent myeloma cell line (SP₂O), available from the American Type Culture Collection, Rockville, Maryland. After fusion, the resulting hybridoma cells are selectively maintained in HAT medium, and then cloned by limiting dilution as described by Wands *et al*. *(Gastroenterology 80*:225-232 (1981)). The hybridoma cells obtained through such a selection are then assayed to identify clones which secrete antibodies capable of binding the connective tissue growth factor-3 protein antigen.

Alternatively, additional antibodies capable of binding to the connective tissue growth factor-3 protein antigen may be produced in a two-step procedure through the use of anti-idiotypic antibodies. Such a method makes use of the fact that antibodies are themselves antigens, and that, therefore, it is possible to obtain an antibody which binds to a second antibody. In accordance with this method, connective tissue growth factor-3-protein specific antibodies are used to immunize an animal, preferably a mouse. The splenocytes of such an animal are then used to produce hybridoma cells, and the hybridoma cells are screened to identify clones which produce an antibody whose ability to bind to the connective tissue growth factor-3 protein-specific antibody can be blocked by the connective tissue growth factor-3 protein antigen. Such antibodies comprise anti-idiotypic antibodies to the connective tissue growth factor-3 protein-specific antibody and can be used to immunize an animal to induce formation of further connective tissue growth factor-3 protein-specific antibodies.

It will be appreciated that Fab and F(ab')₂ and other fragments of the antibodies of the present invention may be used according to the methods disclosed herein. Such fragments are typically produced by proteolytic cleavage, using enzymes such as papain (to produce Fab fragments) or pepsin (to produce F(ab')₂ fragments). Alternatively, connective tissue growth factor-3 protein-binding fragments can be produced through the application of recombinant DNA technology or through synthetic chemistry.

Where *in vivo* imaging is used to detect enhanced levels of connective tissue growth factor-3 protein for tumor diagnosis in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. See, for review, Morrison, *Science 229:*1202 (1985); Oi *et al*., *BioTechniques 4:*214 (1986); Cabilly *et al*., U.S. Patent No. 4,816,567; Taniguchi *et al*., EP 171496; Morrison *et al*., EP 173494; Neuberger *et al*., WO 8601533; Robinson *et al*., WO 8702671; Boulianne *et al*., *Nature 312*:643 (1984); Neuberger *et al., Nature 314:*268 (1985).

Further suitable labels for the connective tissue growth factor-3 protein-specific antibodies of the present invention are provided below. Examples of suitable enzyme labels include malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholine esterase.

Examples of suitable radioisotopic labels include ³H, ¹¹¹In, ¹²⁵I, ¹³¹I, ³²P, ³⁵S, ¹⁴C, ⁵¹Cr, ⁵⁷To, ⁵⁸Co, ⁵⁹Fe, ⁷⁵Se, ¹⁵²Eu, ⁹⁰Y, ⁶⁷Cu, ²¹⁷Ci, ²¹¹At, ²¹²Pb, ⁴⁷Sc, ¹⁰⁹Pd, etc. ¹¹¹In is a preferred isotope where *in vivo* imaging is used since it avoids the problem of dehalogenation of the ¹²⁵I or ¹³¹I-labeled monoclonal antibody by the liver. In addition, this radionucleotide has a more favorable gamma emission energy for imaging (Perkins *et al*., *Eur. J. Nucl. Med. 10*:296-301 (1985); Carasquillo *et al., J. Nucl. Med. 28*:281-287 (1987)). For example, ¹¹¹In coupled to monoclonal antibodies with 1-(P-isothiocyanatobenzyl)-DPTA has shown little uptake in non-tumorous tissues, particularly the liver, and therefore enhances specificity of tumor localization (Esteban *et al., J Nucl. Med. 28*:861-870 (1987)).

Examples of suitable non-radioactive isotopic labels include ¹⁵⁷Gd, ⁵⁵Mn, ¹⁶²Dy, ⁵²Tr, and ⁵⁶Fe.

Examples of suitable fluorescent labels include an ¹⁵²Eu label, a fluorescein label, an isothiocyanate label, a rhodamine label, a phycoerythrin label, a phycocyanin label, an allophycocyanin label, an o-phthaldehyde label, and a fluorescamine label.

Examples of suitable toxin labels include diphtheria toxin, ricin, and cholera toxin.

Examples of chemiluminescent labels include a luminal label, an isoluminal label, an aromatic acridinium ester label, an imidazole label, an acridinium salt label, an oxalate ester label, a luciferin label, a luciferase label, and an aequorin label.

Examples of nuclear magnetic resonance contrasting agents include heavy metal nuclei such as Gd, Mn, and iron.

Typical techniques for binding the above-described labels to antibodies are provided by Kennedy *et al., Clin. Chim. Acta 70*:1-31 (1976), and Schurs *et* *al*., *Clin. Chim. Acta 81*:1-40 (1977). Coupling techniques mentioned in the latter are the glutaraldehyde method, the periodate method, the dimaleimide method, the m-maleimidobenzyl-N-hydroxy-succinimide ester method.

### Therapeutics: Connective Tissue Growth Factor-3 Protein

It will be appreciated by those skilled in the art that individuals with conditions characterized by a decrease in the standard or normal level of connective tissue growth factor-3 activity, can be treated by using a pharmaceutical composition comprising connective tissue growth factor-3 protein. For example, it is believed that individuals in need of wound healing, tissue repair, or increased bone mass (i.e. patients with osteoporosis) would benefit from such treatment. Thus, the invention further provides the use of an effective amount of an isolated connective tissue growth factor-3 polypeptide of the invention, particularly a mature form of the connective tissue growth factor-3, effective to increase the connective tissue growth factor-3 activity level in an individual for the preparation of a pharmaceutical composition for treating an individual in need of an increased level of connective tissue growth factor-3 activity.

The connective tissue growth factor-3 polypeptide composition will be formulated and dosed in a fashion consistent with good medical practice, taking into account the clinical condition of the individual patient (especially the side effects of treatment with connective tissue growth factor-3 polypeptide alone), the site of delivery of the connective tissue growth factor-3 polypeptide composition, the method of administration, the scheduling of administration, and other factors known to practitioners. The "effective amount" of connective tissue growth factor-3 polypeptide for purposes herein is thus determined by such considerations.

As a general proposition, the total pharmaceutically effective amount of connective tissue growth factor-3 polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the connective tissue growth factor-3 polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The key factor in selecting an appropriate dose is the result obtained, as measured by increases in antibody production, increases in splenocyte or thymocyte number, increase in splenic B-cells, etc. The length of treatment needed to observe changes and the interval following treatment for responses to occur appears to vary depending on the desired effect.

Pharmaceutical compositions containing the connective tissue growth factor-3 of the invention may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The connective tissue growth factor-3 polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Patent 3,773,919; EP 58,481), copolymers of L-glutamic acid and gamma-ethyl-L-glutamate (Sidman, U. *et al., Biopolymers 22*:547-556 (1983)), poly (2- hydroxyethyl methacrylate) (R. Langer *et al., J. Biomed. Mater. Res. 15*:167-277 (1981), and R. Langer, *Chem. Tech. 12*:98-105 (1982)), ethylene vinyl acetate (R. Langer *et al., Id*.) or poly-D- (-)-3-hydroxybutyric acid (EP 133,988). Sustained-release connective tissue growth factor-3 polypeptide compositions also include liposomally entrapped connective tissue growth factor-3 polypeptide. Liposomes containing connective tissue growth factor-3 polypeptide are prepared by methods known *per se:* DE 3,218,121; Epstein *et al., Proc. Natl. Acad. Sci. (USA) 82*:3688-3692 (1985); Hwang *et al*., *Proc. Natl. Acad. Sci. (USA) 77*:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal connective tissue growth factor-3 polypeptide therapy.

For parenteral administration, in one embodiment, the connective tissue growth factor-3 polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the connective tissue growth factor-3 polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The connective tissue growth factor-3 polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of connective tissue growth factor-3 polypeptide salts.

Connective tissue growth factor-3 polypeptide to be used for therapeutic administration must be sterile. Sterility is readily accomplished by filtration through sterile filtration membranes (e.g., 0.2 micron membranes). Therapeutic connective tissue growth factor-3 polypeptide compositions generally are placed into a container having a sterile access port, for example, an intravenous solution bag or vial having a stopper pierceable by a hypodermic injection needle.

Connective tissue growth factor-3 polypeptide ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous connective tissue growth factor-3 polypeptide solution, and the resulting mixture is lyophilized. The infusion solution is prepared by reconstituting the lyophilized connective tissue growth factor-3 polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Chromosome Assays

The nucleic acid molecules of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

In certain preferred embodiments in this regard, the cDNA herein disclosed is used to clone genomic DNA of a connective tissue growth factor-3 protein gene. This can be accomplished using a variety of well known techniques and libraries, which generally are available commercially. The genomic DNA then is used for *in situ* chromosome mapping using well known techniques for this purpose. Typically, in accordance with routine procedures for chromosome mapping, some trial and error may be necessary to identify a genomic probe that gives a good *in situ* hybridization signal.

In addition, in some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified portion.

PCR mapping of somatic cell hybrids is a rapid procedure for assigning a particular DNA to a particular chromosome. Using the present invention with the same oligonucleotide primers, sublocalization can be achieved with panels of portions from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization ("FISH") of a cDNA clone to a metaphase chromosomal spread can be used to provide a precise chromosomal location in one step. This technique can be used with probes from the cDNA as short as 50 or 60 bp. For a review of this technique, see Verma *et al., Human Chromosomes: A Manual Of Basic Techniques*, Pergamon Press, New York (1988).

Once a sequence has been mapped to a precise chromosomal location, the physical position of the sequence on the chromosome can be correlated with genetic map data. Such data are found, for example, in V. McKusick, *Mendelian Inheritance In Man*, available on-line through Johns Hopkins University, Welch Medical Library. The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. This assumes 1 megabase mapping resolution and one gene per 20 kb.

Having generally described the invention, the same will be more readily understood by reference to the following examples, which are provided by way of illustration and are not intended as limiting.

### Examples

### Example 1: Expression and Purification of Connective Tissue Growth Factor-3 in E. coli

The bacterial expression vector pQE9 (pD10) is used for bacterial expression in this example. (QIAGEN, Inc., 9259 Eton Avenue, Chatsworth, CA, 91311), pQE9 encodes ampicillin antibiotic resistance ("Amp^{r}") and contains a bacterial origin of replication ("ori"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN, Inc., *supra,* and suitable single restriction enzyme cleavage sites. These elements are arranged such that an inserted DNA fragment encoding a polypeptide expresses that polypeptide with the six His residues (i.e., a "6 X His tag")) covalently linked to the amino terminus of that polypeptide.

The DNA sequence encoding the desired portion connective tissue growth factor-3 protein lacking the hydrophobic leader sequence is amplified from the deposited cDNA clone using PCR oligonucleotide primers which anneal to the amino terminal sequences of the desired portion of the connective tissue growth factor-3 protein and to sequences in the deposited construct 3' to the cDNA coding sequence. Additional nucleotides containing restriction sites to facilitate cloning in the pQE9 vector are added to the 5' and 3' primer sequences, respectively.

For cloning the mature protein, the 5' primer has the sequence 5' CACCACGGATCCAAGGTGCGTACCCAGCTGTGCCCG 3' (SEQ ID NO:4) containing the underlined *Bam*HI restriction site, which encodes 24 nucleotides of the connective tissue growth factor-3 protein coding sequence in Figure 1 (SEQ ID NO:1) beginning immediately after the signal peptide. One of ordinary skill in the art would appreciate, of course, that the point in the protein coding sequence where the 5' primer begins may be varied to amplify a DNA segment encoding any desired portion of the complete connective tissue growth factor-3 protein shorter or longer than the mature form.

The 3' primer has the sequence 5' GATGTAAGCTTCGTGTCCCCATTCCCAGCCCG 3' (SEQ ID NO:5) containing the underlined *Hind*III restriction site followed by 21 nucleotides complementary to the sequence immediately downstream from the connective tissue growth factor-3 protein coding sequence in Figure 1.

The amplified connective tissue growth factor-3 DNA fragment and the vector pQE9 are digested with *Bam*HI and *Hind*III and the digested DNAs are then ligated together. Insertion of the connective tissue growth factor-3 DNA into the restricted pQE9 vector places the connective tissue growth factor-3 protein coding region downstream from the IPTG-inducible promoter and in-frame with an initiating AUG and the six histidine codons.

The ligation mixture is transformed into competent *E. coli* cells using standard procedures, such as those described in Sambrook *et al., Molecular Cloning: a Laboratory Manual,* 2nd Ed.; Cold Spring Harbor Laboratory Press, Cold Spring Harbor, N.Y. (1989). *E. coli* strain M15/rep4, containing multiple copies of the plasmid pREP4, which expresses lac repressor and confers kanamycin resistance ("Kan^{r}"), is used in carrying out the illustrative example described herein. This strain, which is only one of many that are suitable for expressing connective tissue growth factor-3 protein, is available commercially from QIAGEN, Inc., *supra.* Transformants are identified by their ability to grow on LB plates in the presence of ampicillin and kanamycin. Plasmid DNA is isolated from resistant colonies and the identity of the cloned DNA confirmed by restriction analysis, PCR, and DNA sequencing.

Clones containing the desired constructs are grown overnight ("O/N") in liquid culture in LB media supplemented with both ampicillin (100 µg/ml) and kanamycin (25 µg/ml). The O/N culture is used to inoculate a large culture, at a dilution of approximately 1:100 to 1:250. The cells are grown to an optical density at 600nm ("OD600") of between 0.4 and 0.6. Isopropyl-b-D-thiogalactopyranoside ("IPTG") is then added to a final concentration of 1 mM to induce transcription from *lac* repressor sensitive promoters, by inactivating the *lac*I repressor. Cells subsequently are incubated further for 3 to 4 hours. Cells are then harvested by centrifugation.

The cells are then stirred for 3-4 hours at 4°C in 6M guanidine-HCl, pH8. The cell debris is removed by centrifugation, and the supernatant containing the connective tissue growth factor-3 is loaded onto a nickel-nitrilo-tri-acetic acid ("NiNTA") affinity resin column (available from QIAGEN, Inc., *supra*). Proteins with a 6 x His tag bind to the NI-NTA resin with high affinity and can be purified in a simple one-step procedure (for details see: The QIAexpressionist, 1995, QIAGEN, Inc., *supra).* Briefly the supernatant is loaded onto the column in 6 M guanidine-HCl, pH8, the column is first washed with 10 volumes of 6 M guanidine-HCl, pH8, then washed with 10 volumes of 6 M guanidine-HCl pH6, and finally the connective tissue growth factor-3 is eluted with 6 M guanidine-HCl, pH5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH7.4, containing protease inhibitors. The renaturation should be performed over a period of 1.5 hours or more. After renaturation, the proteins can be eluted by the addition of 250 mM imidazole. Imidazole is removed by a final dialyzing step against PBS or 50 mM sodium acetate pH6 buffer plus 200 mM NaCl. The purified protein is stored at 4°C or frozen at -80 °C.

### Example 2: Cloning and Expression of Connective Tissue Growth Factor-3 Protein in a Baculovirus Expression System

In this illustrative example, the plasmid shuttle vector pA2 is used to insert the cloned DNA encoding the complete protein, including its naturally associated secretary signal (leader) sequence, into a baculovirus to express the mature connective tissue growth factor-3 protein, using standard methods as described in Summers *et al*., *A Manual of Methods for Baculovirus Vectors and Insect Cell Culture Procedures,* Texas Agricultural Experimental Station Bulletin No. 1555 (1987). This expression vector contains the strong polyhedrin promoter of the *Autographa californica* nuclear polyhedrosis virus (AcMNPV) followed by convenient restriction sites such as *Bam*HI and *Asp*718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate viable virus that express the cloned polynucleotide.

Many other baculovirus vectors could be used in place of the vector above, such as pAc373, pVL941 and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow *et al., Virology 170*:31-39 (1989).

The cDNA sequence encoding the full length connective tissue growth factor-3 protein in the deposited clone, including the AUG initiation codon and the naturally associated leader sequence shown in Figure 1 (SEQ ID NO:2), is amplified using PCR oligonucleotide primers corresponding to the 5' and 3' sequences of the gene.

The 5' primer has the sequence 5' CGGCAGGATCCGCCATCATGAGAGGCACACCGAAGACCC 3' (SEQ ID NO:6) containing the underlined *Bam*HI restriction enzyme site, an efficient signal for initiation of translation in eukaryotic cells, as described by Kozak, M., *J. Mol. Biol. 196*:947-950 (1987), followed by 22 bases of the sequence of the complete connective tissue growth factor-3 protein shown in Figure 1, beginning with the AUG initiation codon.

The 3' primer has the sequence 5' GATGTGGTACCCGTGTCCCCATTCCCAGCCCG 3' (SEQ ID NO:7) containing the underlined *Asp*718 restriction site followed by 21 nucleotides complementary to the 3' noncoding sequence in Figure 1.

The amplified fragment is isolated from a 1% agarose gel using a commercially available kit ("Geneclean," BIO 101 Inc., La Jolla, Ca.). The fragment is then digested with *Bam*HI and *Asp*718, and again purified on a 1% agarose gel. This fragment is designated herein "F1."

The plasmid is digested with the restriction enzymes *Bam*HI and *Asp*718 and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.). This vector DNA is designated herein "V1."

Fragment F 1 and the dephosphorylated plasmid V 1 are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria are identified that contain the plasmid with the human connective tissue growth factor-3 gene using the PCR method, in which one of the primers that is used to amplify the gene and the second primer is from well within the vector so that only those bacterial colonies containing the connective tissue growth factor-3 gene fragment will show amplification of the DNA. The sequence of the cloned fragment is confirmed by DNA sequencing. This plasmid is designated herein pBacconnective tissue growth factor-3.

Five µg of the plasmid pBacconnective tissue growth factor-3 is cotransfected with 1.0 µg of a commercially available linearized baculovirus DNA ("BaculoGold™ baculovirus DNA", Pharmingen, San Diego, CA.), using the lipofection method described by Felgner *et al., Proc. Natl. Acad. Sci USA 84*:7413-7417 (1987). One µg of BaculoGold™ virus DNA and 5 µg of the plasmid pBacconnective tissue growth factor-3 are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation is continued at 27°C for four days.

After four days the supernatant is collected and a plaque assay is performed, as described by Summers and Smith, *supra*. An agarose gel with "Blue Gal" (Life Technologies Inc., Gaithersburg, MD) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, MD, pages 9-10). After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4°C. The recombinant virus is called V-connective tissue growth factor-3.

To verify the expression of the connective tissue growth factor-3 gene, Sf9 cells are grown in Grace's medium supplemented with 10% heat inactivated FBS. The cells are infected with the recombinant baculovirus V-connective tissue growth factor-3 at a multiplicity of infection ("MOI") of about 2. Six hours later the medium is removed and is replaced with SF900 II medium minus methionine and cysteine (available from Life Technologies Inc., Rockville, MD). If radiolabeled proteins are desired, 42 hours later, 5 µCi of ³⁵S-methionine and 5 µCi ³⁵S-cysteine (available from Amersham) are added. The cells are further incubated for 16 hours and then they are harvested by centrifugation. The proteins in the supernatant as well as the intracellular proteins are analyzed by SDS-PAGE followed by autoradiography (if radiolabeled). Microsequencing of the amino acid sequence of the amino terminus of purified protein may be used to determine the amino terminal sequence of the mature protein and thus the cleavage point and length of the secretory signal peptide.

### Example 3: Cloning and Expression of Connective Tissue Growth Factor-3 in Mammalian Cells

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences, and intervening sequences flanked by donor and acceptor sites for RNA splicing. Highly efficient transcription can be achieved with the early and late promoters from SV40, the long terminal repeats (LTRs) from Retroviruses, e.g. RSV, HTLVI, HIVI, and the early promoter of the cytomegalovirus (CMV). However, cellular signals can also be used (e.g. human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human HeLa 293, H9 and Jurkat cells, mouse NIH3T3 and C127 cells, Cos 1, Cos 7 and CV1, quail QC1-3 cells, mouse L cells, and Chinese hamster ovary cells.

Alternatively, the gene can be expressed in stable cell lines that contain the gene integrated into a chromosome. The co-transfection with a selectable marker such as dhfr, gpt, neomycin, or hygromycin allows the identification and isolation of the transfected cells.

The transfected gene can also be amplified to express large amounts of the encoded protein. Dihydrofolate reductase (DHFR) is a useful marker to develop cell lines that carry several hundred or even several thousand copies of the gene of interest. Another useful selection marker is the enzyme glutamine synthase (GS) (Murphy *et al., Biochem J. 227:*277-279 (1991); Bebbington *et al., Bio*/*Technology 10:*169-175 (1992)). Using these markers, the mammalian cells are grown in selective medium and the cells with the highest resistance are selected. These cell lines contain the amplified gene(s) integrated into a chromosome. Chinese hamster ovary (CHO) cells are often used for the production of proteins.

The expression vectors pC 1 and pC4 contain the strong promoter (LTR) of the Rous Sarcoma Virus (Cullen *et al*., *Molecular and Cellular Biology,* 438-447 (March 1985)) plus a fragment of the CMV-enhancer (Boshart *et al., Cell 41*:521-530 (1985)). Multiple cloning sites, e.g., with the restriction enzyme cleavage sites *Bam*HI, *Aba*I, and *Asp*718, facilitate the cloning of the gene of interest. In addition, vectors contain the 3' intron and the polyadenylation and termination signal of the rat preproinsulin gene.

### Example 3(a): Cloning and Expression in COS Cells

The expression plasmid, pCTGF-3 HA, is made by cloning a cDNA encoding connective tissue growth factor-3 into the expression vector pcDNAI/Amp or pcDNAIII (which can be obtained from Invitrogen, Inc.).

The expression vector pcDNAI/amp contains: (1) an *E.coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron; (5) several codons encoding a hemagglutinin fragment (i.e., an "HA" tag to facilitate purification) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin protein described by Wilson *et al., Cell 37:*767 (1984). The fusion of the HA tag to the target protein allows easy detection and recovery of the recombinant protein with an antibody that recognizes the HA epitope, pcDNAIII contains, in addition, the selectable neomycin marker.

A DNA fragment encoding the connective tissue growth factor-3 protein is cloned into the polylinker region of the vector so that recombinant protein expression is directed by the CMV promoter. The plasmid construction strategy is as follows. The connective tissue growth factor-3 cDNA of the deposited clone is amplified using primers that contain convenient restriction sites, much as described above for construction of vectors for expression of connective tissue growth factor-3 in *E. coli.* Suitable primers include the following, which are used in this example.

The 5' primer, containing the underlined *Bam*HI site, a Kozak sequence, an AUG start codon, and 7 codons of the 5' coding region of the complete connective tissue growth factor-3 has the following sequence:

The 3' primer, containing the underlined *Xba*I site, a stop codon, and 32 bp of 3' coding sequence has the following sequence (at the 3' end):

The PCR amplified DNA fragment and the vector, pcDNAI/Amp, are digested with *Bam*HI and *Xba*I and then ligated. The ligation mixture is transformed into *E. coli* strain SURE (available from Stratagene Cloning Systems, 11099 North Torrey Pines Road, La Jolla, CA 92037). The transformed culture is then plated on ampicillin media plates that are then incubated to allow growth of ampicillin resistant colonies. Plasmid DNA is isolated from resistant colonies and examined by restriction analysis or other means for the presence of the connective tissue growth factor-3-encoding fragment.

For expression of recombinant connective tissue growth factor-3, COS cells are transfected with an expression vector, as described above, using DEAE-DEXTRAN, as described, for instance, in Sambrook *et al., Molecular Cloning: a Laboratory Manual*, Cold Spring Laboratory Press, Cold Spring Harbor, New York (1989). Cells are incubated under conditions for expression of connective tissue growth factor-3 by the vector.

Expression of the connective tissue growth factor-3 HA fusion protein is detected by radiolabelling and immunoprecipitation, using methods described in, for example Harlow *et al., Antibodies: a Laboratory Manual,* 2nd Ed.; Cold Spring Harbor Laboratory Press, Cold Spring Harbor, New York (1988). To this end, two days after transfection, the cells are labeled by incubation for 8 hours in media containing ³⁵S-cysteine. The cells and the media are collected, and the cells washed and then lysed with detergent-containing RIPA buffer: 150 mM NaCl, 1% NP-40, 0.1% SDS, 1% NP-40, 0.5% DOC, 50 mM TRIS, pH 7.5, as described by Wilson *et al*. cited above. Proteins are precipitated from the cell lysate and from the culture media using an HA-specific monoclonal antibody. The precipitated proteins are then analyzed by SDS-PAGE gels and autoradiography. An expression product of the expected size is seen in the cell lysate, which is not seen in negative controls.

### Example 3(b): Cloning and Expression in CHO Cells

The vector pC4 is used for the expression of connective tissue growth factor-3 protein. Plasmid pC4 is a derivative of the plasmid pSV2-dhfr [ATCC Accession No. 37146]. The plasmid contains the mouse DHFR gene under control of the SV40 early promoter. Chinese hamster ovary- or other cells lacking dihydrofolate activity that are transfected with these plasmids can be selected by growing the cells in a selective medium (alpha minus MEM, Life Technologies) supplemented with the chemotherapeutic agent methotrexate (MTX). The amplification of the DHFR genes in cells resistant to methotrexate has been well documented (see, e.g., Alt, F. W., *et al., J. Biol. Chem. 253*:1357-1370 (1978); Hamlin, J.L. and Ma, C., *Biochem. et Biophys. Acta 1097*:107-143 (1990); Page, M.J. and Sydenham, M.A., *Biotechnology 9*:64-68 (1991)). Cells grown in increasing concentrations of MTX develop resistance to the drug by overproducing the target enzyme, DHFR, as a result of amplification of the DHFR gene. If a second gene is linked to the DHFR gene it is usually co-amplified and over-expressed. It is known in the art that this approach may be used to develop cell lines carrying more than 1,000 copies of the amplified gene(s). Subsequently, when the methotrexate is withdrawn, cell lines are obtained that contain the amplified gene integrated into one or more chromosome(s) of the host cell.

For expressing the gene of interest, plasmid pC4 contains the strong promoter of the long terminal repeat (LTR) of the Rous Sarcoma Virus (Cullen *et al., Molecular and Cellular Biology 5(3):*438-447 (March 1985)), plus a fragment isolated from the enhancer of the immediate early gene of human cytomegalovirus (CMV) (Boshart *et al., Cell 41*:521-530 (1985)). Downstream from the promoter are restriction enzyme cleavage sites that allow integration of the genes. Behind these cloning sites, the plasmid contains the 3' intron and polyadenylation site of the rat preproinsulin gene. Other high efficiency promoters can also be used for the expression, e.g., the human β-actin promoter, the SV40 early or late promoters or the long terminal repeats from other retroviruses, e.g., HIV and HTLVI. Clontech's Tet-Off and Tet-On gene expression systems and similar systems can be used to express the connective tissue growth factor-3 in a regulated way in mammalian cells (Gossen, M., & Bujard, H., *Proc. Natl. Acad. Sci. USA 89*: 5547-5551 (1992)). For the polyadenylation of the mRNA, other signals, e.g., from the human growth hormone or globin genes can be used as well. Stable cell lines carrying a gene of interest integrated into the chromosomes can also be selected upon co-transfection with a selectable marker such as gpt, G418, or hygromycin. It is advantageous to use more than one selectable marker in the beginning, e.g., G418 plus methotrexate.

The plasmid pC4 is digested with the restriction enzymes *Bam*HI and *Asp*718, and then dephosphorylated using calf intestinal phosphates by procedures known in the art. The vector is then isolated from a 1% agarose gel.

The DNA sequence encoding the complete connective tissue growth factor-3 protein including its leader sequence is amplified using PCR oligonucleotide primers corresponding to the 5' and 3' sequences of the gene.

The 5' primer has the sequence 5' CGGCAGGATCCGCCATCATGAGAGGCACACCGAAGACCC 3' (SEQ ID NO:6) containing the underlined *Bam*H1 restriction enzyme site followed by an efficient signal for initiation of translation in eukaryotes, as described by Kozak, M., *J. Mol. Biol. 196*:947-950 (1987), and 22 bases of the coding sequence of connective tissue growth factor-3 shown in Figure 1 (SEQ ID NO:1).

The 3' primer has the sequence 5' GATGTGGTACCCGTGTCCCCATTCCCAGCCCG 3' (SEQ ID NO:7) containing the underlined *Asp718* restriction site followed by 21 nucleotides complementary to the non-translated region of the connective tissue growth factor-3 gene shown in Figure 1 (SEQ ID NO:1).

The amplified fragment is digested with the endonucleases *Bam*HI and *Asp*718, and then purified again on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC4 using, for instance, restriction enzyme analysis.

Chinese hamster ovary cells lacking an active DHFR gene are used for transfection. Five µg of the expression plasmid pC4 is cotransfected with 0.5 µg of the plasmid pSV2-neo using lipofectin (Felgner *et al., supra*). The plasmid pSV2-neo contains a dominant selectable marker, the neo gene from Tn5, encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days, single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reverse phase HPLC analysis.

### Example 4: Tissue Distribution of Connective Tissue Growth Factor-3 Protein Expression

Northern blot analysis was carried out to examine connective tissue growth factor-3 gene expression in human tissues, using methods described by, among others, Sambrook *et al*., cited above. A cDNA probe containing the entire nucleotide sequence of the connective tissue growth factor-3 protein (SEQ ID NO:1) was labeled with ³²P using the *redi*prime™ DNA labeling system (Amersham Life Science), according to manufacturer's instructions. After labelling, the probe was purified using a CHROMA SPIN-100™ column (Clontech Laboratories, Inc.), according to manufacturer's protocol number PT1200-1. The purified labelled probe was then used to examine various human tissues for connective tissue growth factor-3 mRNA.

Multiple Tissue Northern (MTN) blots containing various human tissues (H) were obtained from Clontech and were examined with labelled probe using ExpressHyb™ hybridization solution (Clontech) according to manufacturer's protocol number PT1190-1. Following hybridization and washing, the blots were mounted and exposed to film at -70°C overnight, and films developed according to standard procedures.

By Northern expression analysis, CTGF-3 was abundantly expressed in ovary and testis, as well as other organs, as shown below.

| | |
|---|---|
| heart | ++ |
| lung | + |
| skeletal muscle | ++ |
| adrenal medulla | ++ |
| adrenal cortex | +++ |
| testis | +++++ |
| thymus | ++ |
| prostate | +++ |
| testis | +++++ |
| ovary | +++++++ |
| small intestine | + |
| colon | +++ |

It is expected that fibrotic skin or liver would also express high levels of CTGF-3.

It will be clear that the invention may be practiced otherwise than as particularly described in the foregoing description and examples.

## Claims

1. A polynucleotide selected from the group consisting of
(a) polynucleotides encoding the mature form of the polypeptide having the deduced amino acid sequence as shown in Figure 1 [SEQ ID NO:2];
(b) polynucleotides encoding the full length polypeptide having the deduced amino acid sequence as shown in Figure 1 [SEQ ID NO: 2];
(c) polynucleotides encoding the mature form of the polypeptide having the coding sequence as shown in Figure 1 [SEQ ID NO:1];
(d) polynucleotides encoding the full length polypeptide having the coding sequence as shown in Figure 1 [SEQ ID NO: 1];
(e) polynucleotides encoding the polypeptide having the amino acid sequence of the mature form of the polypeptide encoded by the cDNA contained in ATCC 97756;
(f) polynucleotides encoding the polypeptide having the amino acid sequence of the full length polypeptide encoded by the cDNA contained in ATCC 97756;
(g) polynucleotides encoding the mature form of the polypeptide having the coding sequence of the cDNA contained in ATCC 97756;
(h) polynucleotides encoding the full length polypeptide having the coding sequence of the cDNA contained in ATCC 97756;
(i) polynucleotides which are at least 95% identical to a polynucleotide as defined in any one of (a) to (h) and which encode a connective tissue growth factor-3 polypeptide that promotes an increase in bone mass;
(j) polynucleotides encoding a polypeptide having an amino acid sequence at least 95% identical to the amino acid sequence of a connective tissue growth factor-3 polypeptide encoded by the polynucleotide of any one of (a) to (h), wherein said polypeptide promotes an increase in bone mass; and
(k) polynucleotides the complementary strand of which hybridizes under stringent conditions with a polynucleotide of any one of (a) to (h) and encoding a connective tissue growth factor-3 polypeptide wherein said polypeptide promotes an increase in bone mass
or the complementary strand of such a polynucleotide.

2. The polynucleotide of claim 1 which is DNA.

3. The DNA of claim 2 which is genomic DNA.

4. The polynucleotide of claim 1 which is RNA.

5. The polynucleotide of any one of claims 1 to 4 fused to a heterologous polynucleotide.

6. The polynucleotide of claim 5, wherein the heterologous polynucleotide encodes a heterologous polypeptide.

7. The polynucleotide of claim 6, wherein the heterologous polypeptide is fused to a polypeptide encoded by said polynucleotide.

8. A method of making a recombinant vector comprising inserting a polynucleotide of any one of claims 1 to 7 into a vector.

9. A recombinant vector containing the polynucleotide of any one of claims 1 to 7 or produced by the method of claim 8.

10. The vector of claim 9 in which the polynucleotide is operatively linked to expression control sequences allowing expression in prokaryotic or eukaryotic host cells.

11. A method of making a recombinant host cell comprising introducing the vector of claim 9 or 10 into a host cell.

12. A host cell genetically engineered with the polynucleotide of any one of claims 1 to 7 or the vector of claim 9 or 10 or produced by the method of claim 11.

13. The host cell of claim 12, wherein said cell is a prokaryotic cell, eukaryotic cell, animal cell, mammalian cell, insect cell, plant cell, fungal cell, COS cell, CHO cell, or E. coli cell.

14. A process for producing a connective tissue growth factor-3 polypeptide comprising: culturing the host cell of claim 12 or 13 and recovering the polypeptide encoded by said polynucleotide from the culture.

15. A polypeptide having the amino acid sequence encoded by a polynucleotide of any one of claims 1 to 7 or obtainable by the process of claim 14.

16. The polypeptide of claim 15, wherein said polypeptide is a fusion protein.

17. The polypeptide of claim 15 or 16, wherein said polypeptide is fused to a heterologous polypeptide.

18. An antibody specifically binding to the polypeptide of any one of claims 15 to 17.

19. The antibody of claim 18, wherein said antibody is polyclonal, monoclonal, chimeric, single chain, a human antibody, a humanised antibody or an Fab fragment.

20. A pharmaceutical composition comprising the polynucleotide of any one of claims 1 to 7, the polypeptide of any one of claims 15 to 17 or the antibody of claim 18 or 19 or a DNA encoding and capable of expressing said polypeptide in vivo and optionally a pharmaceutically acceptable carrier.

21. A diagnostic composition comprising the polynucleotide of any one of claims 1 to 7 or the antibody of claim 18 or 19.

22. Use of the polypeptide of any one of claims 15 to 17, or the polynucleotide of any one of claims 1 to 7 for the preparation of a pharmaceutical composition for treatment of osteoporosis.

23. An in vitro method of diagnosing osteoporosis comprising:
(a) assaying the expression level of the gene encoding the polypeptide of claim 15 in mammalian cells or body fluid; and
(b) comparing said gene expression level with a standard gene expression level, whereby a decrease in said gene expression level compared to said standard is indicative of osteoporosis.

## Patentansprüche

1. Polynucleotid, ausgewählt aus der Gruppe bestehend aus:
(a) Polynucleotiden, die die reife Form des Polypeptides codieren, welches die abgeleitete Aminosäuresequenz wie in Figur 1 (SEQ ID Nr. 2) gezeigt hat;
(b) Polynucleotiden, die das Volllängen-Polypeptid codieren, welches die abgeleitete Aminosäuresequenz wie in Figur 1 (SEQ ID Nr.: 2) gezeigt hat;
(c) Polynucleotiden, die die reife Form des Polypeptids codieren, welches die codierende Sequenz wie in Figur 1 (SEQ ID Nr.: 1) gezeigt hat;
(d) Polynucleotiden, die das Volllängen-Polypeptid codieren, welches die codierende Sequenz wie in Figur 1 (SEQ ID Nr.: 1) gezeigt hat;
(e) Polynucleotiden, die das Polypeptid codieren, welches die Aminosäuresequenz der reifen Form des Polypeptids hat, welches von der in ATCC 97756 enthaltenen cDNA codiert wird;
(f) Polynucleotiden, die das Polypeptid codieren, welches die Aminosäuresequenz des Volllängen-Polypeptids hat, welches von der in ATCC 97756 enthaltenen cDNA codiert wird;
(g) Polynucleotiden, die die reife Form des Polypeptids codieren, welches die codierende Sequenz der in ATCC 97756 enthaltenen cDNA hat;
(h) Polynucleotiden, die das Volllängen-Polypeptid codieren, welches die codierende Sequenz der in ATCC 97756 enthaltenen cDNA hat;
(i) Polynucleotiden, die zumindestens 95 % identisch sind mit einem Polynucleotid nach einem der Punkte (a) bis (h), und welche ein Bindegewebe-Wachstumsfaktor-3-Polypeptid codieren, das eine Zunahme der Knochenmasse fördert;
(j) Polynucleotiden, die ein Polypeptid codieren, das eine Aminosäuresequenz hat, die zumindestens 95 % identisch ist mit der Aminosäuresequenz eines Bindegewebe-Wachstumsfaktor-3-Polypeptids, welches von dem Polynucleotid nach einem der Punkte (a) bis (h) codiert wird, wobei das Polypeptid eine Zunahme der Knochenmasse fördert; und
(k) Polynucleotiden, deren komplementärer Strang unter stringenten Bedingungen mit einem Polynucleotid nach einem der Punkte (a) bis (h) hybridisiert und die ein Bindegewebe-Wachstumsfaktor-3-Polypeptid codieren, wobei das Polypeptid eine Zunahme der Knochenmasse fördert;
oder der komplementäre Strang eines solchen Polynucleotids.

2. Polynucleotid nach Anspruch 1, welches DNA ist.

3. DNA nach Anspruch 2, welche genomische DNA ist.

4. Polynucleotid nach Anspruch 2, welches RNA ist.

5. Polynucleotid nach einem der Anspruche 1 bis 4, welches an ein heterologes Polynucleotid fusioniert ist.

6. Polynucleotid nach Anspruch 5, wobei das heterologe Polynucleotid ein heterologes Polypeptid codiert.

7. Polynucleotid nach Anspruch 6, wobei das heterologe Polypeptid an ein Polypeptid fusioniert ist, welches von dem Polynucleotid codiert wird.

8. Verfahren zur Herstellung eines rekombinanten Vektors, umfassend die Einführung eines Polynucleotids nach einem der Ansprüche 1 bis 7 in einen Vektor.

9. Rekombinanter Vektor, welcher das Polynucleotid nach einem der Ansprüche 1 bis 7 enthält oder gemäß dem Verfahren nach Anspruch 8 hergestellt wird.

10. Vektor nach Anspruch 9, wobei das Polynucleotid funktionell verknüpft ist mit Expressions-Kontrollsequenzen, welche die Expression in prokaryontischen oder eurkaryontischen Wirtszellen erlauben.

11. Verfahren zur Herstellung einer rekombinanten Wirtszelle, umfassend die Einführung des Vektors nach einem der Ansprüche 9 oder 10 in eine Wirtszelle.

12. Wirtszelle, gentechnisch verändert mit dem Polynucleotid nach einem der Ansprüche 1 bis 7 oder dem Vektor nach Anspruch 9 oder 10 oder hergestellt gemäß dem Verfahren nach Anspruch 11.

13. Wirtszelle nach Anspruch 12, wobei die Zelle eine prokaryontische Zelle, eine eukaryotische Zelle, eine tierische Zelle, eine Säugerzelle, eine Insektenzelle, eine Pflanzenzelle, eine Pilzzelle, eine COS-Zelle, eine CHO-Zelle oder eine E. coli-Zelle ist.

14. Verfahren zur Herstellung eines Bindegewebe-Wachstumsfaktor-3-Polypeptids, umfassend die Züchtung der Wirtszelle nach Anspruch 12 oder 13 und die Gewinnung des Polypeptids, welches von dem Polynucleotid codiert wird, aus der Kultur.

15. Polypeptid, das die Aminosäuresequenz, welche durch ein Polynucleotid nach einem der Ansprüche 1 bis 7 codiert wird, hat oder das gemäß dem Verfahren nach Anspruch 14 erhältlich ist.

16. Polypeptid nach Anspruch 15, wobei das Polypeptid ein Fusionsprotein ist.

17. Polypeptid nach Anspruch 15 oder 16, wobei das Polypeptid an ein heterologes Polypeptid fusioniert ist.

18. Antikörper, welcher spezifisch an das Polypeptid nach einem der Ansprüche 15 bis 17 bindet.

19. Antikörper nach Anspruch 18, wobei der Antikörper ein polyclonaler Antikörper, ein monoclonaler Antikörper, ein chimärer Antikörper, ein Einzelkettenantikörper, ein menschlicher Antikörper, ein humanisierter Antikörper oder ein Fab-Fragment ist.

20. Arzneimittel, umfassend das Polynucleotid nach einem der Ansprüche 1 bis 7, das Polypeptid nach einem der Ansprüche 15 bis 17, oder den Antikörper nach Anspruch 18 oder 19, oder DNA, welche das Polypeptid codiert und in der Lage ist, es in vivo zu exprimieren, und gegebenenfalls einen pharmazeutisch verträglichen Träger.

21. Diagnosemittel, umfassend das Polynucleotid nach einem der Ansprüche 1 bis 7 oder den Antikörper nach Anspruch 18 oder 19.

22. Verwendung des Polypeptids nach einem der Ansprüche 15 bis 17 oder des Polynucleotids nach einem der Ansprüche 1 bis 7 für die Herstellung eines Arzneimittels zur Behandlung von Osteoporose.

23. In vitro-Verfahren zur Diagnose von Osteoporose, umfassend:
(a) Untersuchen der Expressionshöhe des Genes, welches das Polypeptid nach Anspruch 15 codiert, in Säugerzellen oder in Körperflüssigkeit; und
(b) Vergleichen der Genexpressionshöhe mit einer Standardgenexpressionshöhe, wobei eine Abnahme der Genexpressionshöhe im Vergleich zum Standard Osteoporose angezeigt.

## Revendications

1. Polynucléotide sélectionné dans le groupe consistant en
(a) polynucléotides codant pour la forme mature du polypeptide ayant la séquence déduite d'acides aminés telle que montrée à la Figure 1 [SEQ ID NO:2];
(b) polynucléotides codant pour le polypeptide de pleine longueur ayant la séquence déduite d'acides aminés telle que montrée à la Figure 1 [SEQ ID NO:2];
(c) polynucléotides codant pour la forme mature du polypeptide ayant la séquence de codage telle que montrée à la Figure 1 [SEQ ID NO:1];
(d) polynucléotides codant pour le polypeptide de pleine longueur ayant la séquence de codage telle que montrée à la Figure 1 [SEQ ID NO:1];
(e) polynucléotides codant pour le polypeptide ayant la séquence d'acides aminés de la forme mature du polypeptide pour lequel code l'ADNc contenu dans ATCC 97756;
(f) polynucléotides codant pour le polypeptide ayant la séquence d'acides aminés du polypeptide de pleine longueur pour lequel code l'ADNc contenu dans ATCC 97756;
(g) polynucléotides codant pour la forme mature du polypeptide ayant la séquence de codage de l'ADNc contenu dans ATCC 97756;
(h) polynucléotides codant pour le polypeptide de pleine longueur ayant la séquence de codage de l'ADNc contenu dans ATCC 97756;
(i) polynucléotides qui sont identiques à au moins 95% avec un polynucléotide tel que défini dans l'un quelconque de (a) à (h), et qui codent pour un polypeptide du facteur 3 de croissance du tissu conjonctif pour lequel code le polynucléotide de l'un quelconque de (a) à (h) où ledit polypeptide favorise une augmentation de la masse osseuse;
(j) polynucléotides codant pour un polynucléotide ayant une séquence d'acides aminés qui est identique à au moins 95% avec la séquence d'acides aminés d'un polypeptide du facteur 3 de croissance du tissu conjonctif qui est codé par le polynucléotide de l'un quelconque de (a) à (h) où ledit polypeptide favorise une augmentation de la masse osseuse; et
(k) polynucléotides dont le brin complémentaire s'hybride en conditions stringentes avec un polynucléotide de l'un quelconque de (a) à (h) et codant pour un polypeptide du facteur 3 de croissance du tissu conjonctif, où ledit polypeptide favorise une augmentation de la masse osseuse;
ou le brin complémentaire d'un tel polynucléotide.

2. Polynucléotide de la revendication 1 qui est un ADN.

3. ADN de la revendication 2 qui est un ADN génomique.

4. Polynucléotide de la revendication 2 qui est un ARN.

5. Polynucléotide de l'une quelconque des revendications 1 à 4 fusionné à un polynucléotide hétérologue.

6. Polynucléotide de la revendication 5, où le polynucléotide hétérologue code pour un polypeptide hétérologue.

7. Polynucléotide de la revendication 6, où le polypeptide hétérologue est fusionné à un polypeptide pour lequel code ledit polynucléotide.

8. Méthode de fabrication d'un vecteur recombinant comprenant l'insertion d'un polynucléotide de l'une quelconque des revendications 1 à 7 dans un vecteur.

9. Vecteur recombinant contenant le polynucléotide de l'une quelconque des revendications 1 à 7 ou produit par la méthode de la revendication 8.

10. Vecteur de la revendication 9 dans lequel le polynucléotide est opérativement enchaîné à des séquences de contrôle d'expression permettant l'expression dans des cellules hôtes procaryotes ou eucaryotes.

11. Méthode de fabrication d'une cellule hôte recombinante, comprenant l'introduction du vecteur de la revendication 9 ou 10 dans une cellule hôte.

12. Cellule hôte génétiquement manipulée avec le polynucléotide de l'une quelconque des revendications 1 à 7 ou le vecteur de la revendication 9 ou 10 ou bien produite par la méthode de la revendication 11.

13. Cellule hôte de la revendication 12, où ladite cellule est une cellule procaryote, une cellule eucaryote, une cellule animale, une cellule mammalienne, une cellule d'insecte, une cellule de plante, une cellule fongique, une cellule COS, une cellule CHO, ou une cellule de E. coli.

14. Procédé de production d'un polypeptide d'un facteur 3 de croissance du tissu conjonctif comprenant: la culture de la cellule hôte de la revendication 12 ou 13 et la récupération du polypeptide pour lequel code ledit polynucléotide, de la culture.

15. Polypeptide ayant la séquence d'acides aminés pour laquelle code un polypeptide de l'une quelconque des revendications 1 à 7 ou bien pouvant être obtenu par le procédé de la revendication 14.

16. Polypeptide de la revendication 15, où ledit polypeptide est une protéine de fusion.

17. Polypeptide de la revendication 15 ou 16, où ledit polypeptide est fusionné à un polypeptide hétérologue.

18. Anticorps se liant spécifiquement au polypeptide de l'une quelconque des revendications 15 à 17.

19. Anticorps de la revendication 18, où ledit anticorps est un anticorps polyclonal, monoclonal, chimérique, monocaténaire humain, un anticorps humanisé ou un fragment Fab.

20. Composition pharmaceutique comprenant le polynucléotide de l'une quelconque des revendications 1 à 7, le polypeptide de l'une quelconque des revendications 15 à 17, où l'anticorps de la revendication 18 ou 19 ou un ADN codant pour et capable d'exprimer ledit polypeptide in vivo et facultativement un support pharmaceutiquement acceptable.

21. Composition de diagnostic comprenant le polynucléotide de l'une quelconque des revendications 1 à 7, ou l'anticorps de la revendication 18 ou 19.

22. Utilisation d'un polypeptide de l'une quelconque des revendications 15 à 17, ou bien du polynucléotide de l'une quelconque des revendications 1 à 7 pour la préparation d'une composition pharmaceutique pour le traitement de l'ostéoporose.

23. Méthode in vitro pour le diagnostic de l'ostéoporose comprenant:
(a) l'évaluation du niveau d'expression du gène codant pour le polypeptide de la revendication 15 dans des cellules mammaliennes ou un fluide corporel; et
(b) la comparaison dudit niveau d'expression du gène avec un niveau d'expression d'un gène standard et une diminution du niveau d'expression dudit gène comparée audit standard indique l'ostéoporose.
